# EUROPÄISCHE PATENTSCHRIFT

(11) **EP 3 994 202 B1**
(45) Veröffentlichungstag und Bekanntmachung des Hinweises auf die Patenterteilung: **12.08.2026**
(21) Anmeldenummer: 20734693.3
(22) Anmeldetag: 23.06.2020
(51) Int. Cl.: C08J 3/075, C08J 3/24, C08L 71/02, C08L 101/02, A61K 47/00, A61K 9/06

(54) **NEUARTIGE HYDROGELE**
NOVEL HYDROGELS
HYDROGELS NOVATEURS

(30) Priorität: 03.07.2019 DE 102019117997
(43) Veröffentlichungstag der Anmeldung: 11.05.2022
(73) Patentinhaber: INM - Leibniz-Institut für Neue Materialien gemeinnützige GmbH, 66123 Saarbrücken (DE)
(72) Erfinder: PAEZ, Julieta I., 66123 Saarbrücken (DE); FARRUKH, Aleeza, Irvine, California 92617 (US); DEL CAMPO BÉCARES, Maria Aránzazu, 55128 Mainz-Bretzenheim (DE)
(74) Vertreter: Patentanwälte Gierlich & Pischitzis Partnerschaft mbB
(86) Internationale Anmeldenummer: PCT/EP2020/067410
(87) Internationale Veröffentlichungsnummer: WO 2021/001203

(56) Entgegenhaltungen:
- KHARKAR PRATHAMESH M. ET AL: "Design of thiol- and light-sensitive degradable hydrogels using Michael-type addition reactions", POLYMER CHEMISTRY, vol. 6, no. 31, 28 July 2015 (2015-07-28), Cambridge, pages 5565 - 5574, XP055979147, ISSN: 1759-9954, DOI: 10.1039/C5PY00750J
- PRATHAMESH M. KHARKAR ET AL: "Thiol-ene Click Hydrogels for Therapeutic Delivery", ACS BIOMATERIALS SCIENCE & ENGINEERING, vol. 2, no. 2, 25 January 2016 (2016-01-25), US, pages 165 - 179, XP055611460, ISSN: 2373-9878, DOI: 10.1021/acsbiomaterials.5b00420
- EDWARD A. PHELPS ET AL: "Maleimide Cross-Linked Bioactive PEG Hydrogel Exhibits Improved Reaction Kinetics and Cross-Linking for Cell Encapsulation and In Situ Delivery", ADVANCED MATERIALS, vol. 24, no. 1, 16 December 2011 (2011-12-16), pages 64 - 70, XP055539198, ISSN: 0935-9648, DOI: 10.1002/adma.201103574
- ALEEZA FARRUKH ET AL: "Bioconjugating Thiols to Poly(acrylamide) Gels for Cell Culture Using Methylsulfonyl Co-monomers", ANGEWANDTE CHEMIE, vol. 55, no. 6, 5 February 2016 (2016-02-05), DE, pages 2092 - 2096, XP055536746, ISSN: 1433-7851, DOI: 10.1002/anie.201509986
- NARIHIRO TODA ET AL: "Rapid, Stable, Chemoselective Labeling of Thiols with Julia-Kocieński-like Reagents: A Serum-Stable Alternative to Maleimide-Based Protein Conjugation", ANGEWANDTE CHEMIE, vol. 52, no. 48, 2 October 2013 (2013-10-02), DE, pages 12592 - 12596, XP055416940, ISSN: 1433-7851, DOI: 10.1002/anie.201306241

## Beschreibung

### Gebiet der Erfindung

Die Erfindung betrifft Hydrogele, Verfahren zu ihrer Herstellung sowie ihre Verwendung.

### Stand der Technik

Hydrogele sind dreidimensionale Netzwerke von vernetzten hydrophilen Polymeren, welche einen hohen Anteil an Wasser enthalten. Solche Materialien sind bekannt als Matrixmaterialien für biologische Anwendungen wie Wirkstofftransport, Wundmaterialien, Gewebezüchtung und können auch in der Zellkultur verwendet werden. Durch ihre wässrige und poröse Struktur erlauben sie einen guten Transport von Nährstoffen zu den Zellen.

Viele natürliche oder synthetische Polymere wurden bereits zur Herstellung von Hydrogelen verwendet, beispielsweise Collagen, Gelatine, Polyethylenglykol (PEG). Zur Vernetzung der Hydrogele wurden unterschiedliche Reaktionen und Mechanismen untersucht, beispielsweise Photopolymerisation, Michaeladdition oder ähnliche.

Gerade die Steuerung der Vernetzungsreaktion ist eine große Herausforderung. Insbesondere wenn das Hydrogel zur Umhüllung von Zellen erzeugt werden soll. Polymerisiert das Gel zu schnell, ist es häufig nicht homogen vernetzt. Wenn es zu langsam polymerisiert, können sich die einzuschließenden Bestandteile, z. B. Zellen, ablagern und werden nicht homogen eingeschlossen

P. M. Kharkar, et al., HHS Author Manuscripts, 2016, 2, 165-179, beschreibt Hydrogele, die durch Reaktion von mehrarmigen Poly(ethylenglykol)-Makromonomeren, die mit Alkenylgruppen oder Maleimidgruppen funktionalisiert sind und mehrarmigen Poly(ethylenglykol) -Makromonomeren, die mit Thiolen funktionalisiert sind, hergestellt werden.

E. A. Phelps et al., Advanced Materials, 2011, 24, 64-50, beschreibt Hydrogele, die durch Reaktion eines 4-armigen PEG-Maleimid-Makromers, eines 4-armigen PEG-Acrylat-Makromers oder eines 4-armigen PEG-Vinylsulfon-Makromers mit einem thiolhaltigen Peptid hergestellt werden.

P. M. Kharkar, et al., Polymer Chemistry, 2015, 6, 5565-5574, beschreibt ein Hydrogel, das durch die Reaktion eines mit Arylthiolen funktionalisierten vierarmigen Polyethylenglykols (PEG-4-arylSH) mit einem mit Maleinimiden funktionalisierten vierarmigen Polyethylenglykol (PEG-4-PD-MI) durch eine Additionsreaktion vom Michael-Typ erhalten wird.

N.Toda, et al., Angew. Chem. Int. Ed, 2013, 52, 12592-12596, beschreibt die nukleophile Substitution von Sulfonylgruppen in heteroaromatischen Gruppen durch Thiolgruppen, aber nicht diese Reaktion zur Vernetzung zur Herstellung von Hydrogelen.

### Aufgabe

Aufgabe der Erfindung ist es, ein Verfahren zur Herstellung eines Hydrogels bereitzustellen, welches eine Verwendung insbesondere zur Umhüllung von Zellen erlaubt. Es ist außerdem Aufgabe, ein entsprechendes Hydrogel und seine Verwendung bereitzustellen.

### Lösung

Diese Aufgabe wird durch die Erfindungen mit den Merkmalen der unabhängigen Ansprüche gelöst. Vorteilhafte Weiterbildungen der Erfindungen sind in den Unteransprüchen gekennzeichnet. Der Wortlaut sämtlicher Ansprüche wird hiermit durch Bezugnahme zum Inhalt dieser Beschreibung gemacht. Die Erfindungen umfassen auch alle sinnvollen und insbesondere alle erwähnten Kombinationen von unabhängigen und/oder abhängigen Ansprüchen. Verfahren zur Herstellung eines Hydrogels umfassend folgende Schritte:
a) Herstellen einer Zusammensetzung umfassend
   a1) mindestens ein Makromer umfassend als funktionelle Gruppen mindestens zwei Thiolgruppen,
   a2) mindestens ein Makromer umfassend als funktionelle Gruppen mindestens zwei aromatische oder heteroaromatische Gruppen, die jeweils mit mindestens einer Sulfonylgruppe substituiert sind, wobei mindestens eine Komponente a1) oder a2) mindestens drei der genannten funktionellen Gruppen aufweist;
b) Reaktion der beiden Makromere über die funktionellen Gruppen unter Bildung eines Hydrogels.

Im Folgenden werden einzelne Verfahrensschritte näher beschrieben. Die Schritte müssen nicht notwendigerweise in der angegebenen Reihenfolge durchgeführt werden, und das zu schildernde Verfahren kann auch weitere, nicht genannte Schritte aufweisen.

Unter einem Makromer wird eine Verbindung verstanden, welche eine mittlere molare Masse von unter 500 kDa aufweist, bevorzugt von unter 100 kDa, insbesondere von unter 50 kDa. Die mittlere molare Masse wird als gewichtsmittleres Molekulargewicht mit Gel-Permeations-Chromatographie (GPC) bestimmt.

Besonders bevorzugt sind Makromere mit einer mittleren molaren Masse von unter 50 kDa, insbesondere von unter 30 kDa.

In einer besonderen Ausführungsform der Erfindung liegt die mittlere molare Masse eines Makromers zwischen 100 Da und 500 kDa, bevorzugt zwischen 200 Da und 200 kDa, insbesondere zwischen 800 Da und 100 kDa.

Wichtig ist dabei, dass das Makromer die entsprechenden funktionellen Gruppen trägt und diese zur Reaktion zur Verfügung stehen.

Bevorzugt sind dabei Makromere, welche **2, 3, 4,** 5, 6, 7, 8, **9** oder 10 funktionelle Gruppen aufweisen, bevorzugt **2, 3, 4,** 5, 6, 7, 8 funktionelle Gruppen, besonders bevorzugt **2, 3, 4, 5** oder 6 funktionelle Gruppen, insbesondere **2,** 3 oder 4 funktionelle Gruppen aufweisen.

Unter Bildung des Hydrogels bedeutet, dass durch die Vernetzung ein Hydrogel gebildet wird. Es finden also ausreichend Vernetzungsreaktionen statt. Dies kann durch die Art und Menge der eingesetzten Komponenten gesteuert werden.

In einer weiteren bevorzugten Ausführungsform weist mindestens eine Komponente a1) oder a2) mindestens 4 der genannten funktionellen Gruppen auf.

In einer bevorzugten Ausführungsform weisen beide Komponenten a1) und a2) mindestens **3,** bevorzugt mindestens **4,** der genannten funktionellen Gruppen auf. Besonders bevorzugt weisen beide Komponenten a1) und a2) **3, 4,** 5, 6, 7, 8, 9 oder 10 funktionelle Gruppen auf, bevorzugt **3, 4,** 5, 6, 7, 8 funktionelle Gruppen, besonders bevorzugt **3, 4,** 5 oder 6 funktionelle Gruppen, insbesondere 3 oder 4 funktionelle Gruppen.

Bevorzugt sind wasserlösliche Makromere. Dies bedeutet, dass die Makromere in notwendigem Maße unter den Bedingungen der Reaktion in Lösung vorliegen.

Bevorzugt sind Makromere basierend auf Oligomeren oder Polymeren. Es können natürliche oder künstliche Oligomere oder Polymere sein. Beispiele für künstliche Oligomere oder Polymere sind Poly(meth)acrylate wie Poly(meth)acrylamide, Po-ly(meth)acrylsäure, PolyHPMA oder PolyHEMA, Polyethylenglykol (PEG), Polyvinylalkohol (PVA), Polyurethan (PU), Polyvinylpyrrolidon (PVP), Polyamide, Poly(amidoamine) (PAMAM), Polyester, Polylactide, Polyglycolsäure (PGA) oder Poly(lactid-co-glycolid) (PLGA), Polyanhydride, Poly(ortho)ester, Polyacetale, Poloxamere (Blockcopolymere aus Ethylenoxid (PEG) und Propylenoxid (PPG)) wie PEG-Co-PPG-Co-PEG), Poly-2-oxazoline, Polyphosphazene, Polyglycerin, Polyamine wie Polylysin oder Polyethylenimin (PEI), Polycarbonate, Polyglutaminsäure, insbesondere Poly-Gamma-Glutaminsäure, Polyasparaginsäure (PASA), Polyphosphonate, oder natürlichen Oligomeren wie DNA, RNA, Gelatine, Polyhydroxyalkanoate (PHA), Poly-Gamma-Glutaminsäure, Proteine oder Peptide wie Kollagene, VPM, Albumin oder Fibrin, Polysacccharide wie Agarose, Chitin, Chitosan, Chondroitin, Mannan, Inulin, Dextran, Cellulose, Alginate oder Hyaluronsäure. Bevorzugt sind Oligomere basierend auf Polyethylenglykol. Die Oligomere und Polymere sind mit den entsprechenden funktionellen Gruppen funktionalisiert.

Im Falle der Oligomere auf Peptidbasis werden die Thiolgruppen bevorzugt durch die entsprechenden Aminosäuren wie Cystein oder Homocystein bereitgestellt. Auf Peptidbasis bedeutet dabei, dass das entsprechende Oligomer zu mindestens 80 % der molekularen Masse aus natürlichen oder unnatürlichen Aminosäuren aufgebaut ist. Solche Oligomere weisen daher mindestens zwei Thiolgruppen auf, insbesondere mindestens zwei Cystein.

Die zumindest teilweise Verwendung von natürlichen Polymeren erlaubt auch die Einführung von spezifisch spaltbaren Stellen im Hydrogel, beispielsweise durch Enzyme.

Es kann erforderlich sein, dass die funktionellen Gruppen über einen kurzen Linker an das Oligomer oder Polymer gebunden werden, beispielsweise über eine oder mehrere Ester, Ether oder Amidbindungen. Bevorzugt sind Linker mit einer molaren Masse von unter 1500 mol, bevorzugt von unter 800 mol, insbesondere unter 500 mol oder unter 200 mol.

Die Thiolgruppen liegen bevorzugt als freie Thiolgruppen vor. Es ist auch möglich, dass sie mit Gruppen versehen sind, welche vor der Bildung des Hydrogels abgespalten werden.

Das Makromer a2) ist ein Makromer umfassend mindestens zwei aromatische Gruppen, die jeweils mit mindestens einer Sulfonylgruppe substituiert sind. Dies sind Gruppen der Formel (1) :

M-Ar-SO₂-R¹ (1)

wobei Ar für eine elektronenarme Arylgruppe oder elektronenarme Heteroarylgruppe steht. Dadurch ist es möglich Reaktionsbedingungen zu wählen, bei welchen die Thiolgruppen des ersten Makromers eine nukleophile aromatische Substitution an der Gruppe Ar durchführen können, wobei die Gruppe SO₂-R¹ als Abgangsgruppe dient.

M steht für eine kovalente Verbindung zum Makromer und ist bevorzugt eine Einfachbindung, Ether, oder Carbonylgruppe. Die Carbonylgruppe kann Teil einer Ester oder Amidbindung sein. So können als Gruppe Ar die entsprechenden Ester oder Amide zur Ankopplung an das Makromer verwendet werden, wie beispielsweise entsprechend substituierte Benzoesäureester oder Benzoesäureamide.

Eine Arylgruppe im Sinne dieser Erfindung enthält 6 bis 40 C-Atome; eine Heteroarylgruppe im Sinne dieser Erfindung enthält 1 bis 40 C-Atome und mindestens ein Heteroatom, mit der Maßgabe, dass die Summe aus C Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Naphthalimid, Anthracen, Chinolin, Isochinolin, etc., verstanden.

Unter einer elektronenarmen Arylgruppe oder Heteroarylgruppe wird eine Arylgruppe oder Heteroarylgruppe verstanden, dessen n-Elektronendichte durch negative Induktionseffekte oder negative Mesomerieeffekte (-I-Effekte bzw. -M-Effekte) verringert ist. Eine Aufstellung von Substituenten oder Gruppen, die diese Effekte hervorrufen, findet sich in jedem Standardlehrbuch der organischen Chemie. Als Beispiele seien ohne Beschränkung genannt für -I-Substituenten: OH, Halogene, insbesondere Fluor und Chlor, NO₂, ungesättigte Gruppen; für -M-Substituenten: NO₂, CN, Arylgruppen oder Heteroarylgruppen. Diese elektronenziehenden Gruppen (EWG, engl. "electron withdrawing groups") müssen natürlich in Konjugation zur Abgangsgruppe -SO₂-R¹ stehen, d.h. bei Carbocyclen in ortho- oder para-Stellung, um den gewünschten Effekt ausüben zu können. Im Falle von Heteroarylgruppen tragen die Heteroatome entsprechend abhängig von ihrer Position zur Verringerung der Elektronendichte bei. Es können auch mehrere verschiedene Gruppen vorhanden sein.

Im Falle von elektronenarmen Arylgruppen ist Ar ausgewählt aus der Gruppe umfassend Nitrobenzole, Benzaldehyde, Benzonitrile, Benzoesäureester, welche noch mit einer oder mehreren Gruppen R² wie nachstehend definiert substituiert sein können. Beispiel für eine solche Arylgruppe sind Verbindungen auf der Grundlage von Nitrobenzoesäure mit 1 oder 2 Nitrogruppen, beispielsweise Nitrobenzoesäureester oder Nitrobenzoesäureamide, welche mindestens an einer Position eine
-SO₂-R¹-Gruppe aufweisen. Bevorzugt ist diese Gruppe in Metaposition zu einer Nitrogruppe angeordnet. Besonders bevorzugt ist eine Nitrogruppe in 3-Position und die -SO₂-R¹-Gruppe in 4-Position. Beispiele für eine solche Verbindung ist 3-Nitro-4-Sulfomethyl-Benzoesäure.

Im Falle von elektronenarmen Heteroarylgruppen ist Ar ausgewählt aus der Gruppe umfassend einkernige Heteroaromaten wie Pyridine, Pyrimidine, Pyrazine, Pyridazine, Triazine, wie 1,3,5-Triazin, 1,2,4-Triazin oder 1,2,3-Triazin, Tetrazine, wie 1,2,4,5-Tetrazin, 1,2,3,4-Tetrazin oder 1,2,3,5-Tetrazin, Oxazole, Isooxazol, Thiazole, wie 1,2-Thiazol oder 1,3-Thiazol, Isothiazol, Oxadiazole, wie 1,2,3-Oxadiazol, 1,2,4-Oxadiazol, 1,2,5-Oxadiazol und 1,3,4-Oxadiazol, Thiadiazole, wie 1,2,3-Thiadiazol, 1,2,4-Thiadiazol, 1,2,5-Thiadiazol oder 1,3,4-Thiadiazol, Imidazol, Pyrazol, Triazole, wie insbesondere 1,2,4-Triazol oder 1,2,3-Triazol, Tetrazol, mehrkernige Heteroaromaten, wie Chinoline, Isochinoline, Naphthalimid, Benzimidazol, Benzoxazol, Benzothiazol, Benzopyridazin, Benzpyrimidin, Chinoxalin, Benzotriazol, Purin, Pteridin, Indolizin und Benzothiadiazol, welche noch mit einer oder mehreren Gruppen R² wie nachstehend definiert substituiert sein können.

Bevorzugte Heteroarylgruppen sind Oxadiazole und Benzothiazol. In einer weiteren besonders bevorzugten Ausführungsform ist Ar eine Oxadiazolgruppe, insbesondere eine 1,3,4-Oxadiazolgruppe, welche bevorzugt mit mindestens einer Phenylgruppe substituiert ist, insbesondere mit einer Phenylgruppe.

R¹ steht für N(R²)₂, eine geradkettige Alkylgruppe mit 1 bis 20 C-Atomen oder eine verzweigte oder cyclische Alkylgruppe mit 3 bis 20 C-Atomen, oder eine Alkenyl- oder Alkinylgruppe mit 2 bis 20 C-Atomen , wobei die Alkyl-, Alkenyl- oder Alkinylgruppe jeweils mit einem oder mehreren Resten R² substituiert sein kann und wobei eine oder mehrere nicht benachbarte CH₂-Gruppen durch O, NR², S, R²C=CR², C=C, C=O, C(=O)O oder C(=O)NR² ersetzt sein können, oder eine Arylgruppe oder Heteroarylgruppe, die jeweils mit einem oder mehreren Resten R² substituiert sein kann.

R² ist bei jedem Auftreten gleich oder verschieden H, D, F, Cl, Br, I, N(R³)₂, CN, NO₂, OR³, SR³, C(=O)OR³, C(=O)N(R³)₂, C(=O)R³, eine geradkettige Alkylgruppe mit 1 bis 20 C Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R³ substituiert sein kann, wobei eine oder mehrere nicht benachbarte CH₂-Gruppen durch R³C=CR³, C=C, C=O, NR³, O, S, C(=O)O oder C(=O)NR³ ersetzt sein können, oder eine Arylgruppe oder Heteroarylgruppe, die jeweils mit einem oder mehrere Resten R³ substituiert sein kann.

R³ ist bei jedem Auftreten gleich oder verschieden H, D, F, OH, oder ein aliphatischer, aromatischer und/oder heteroaromatischer organischer Rest, insbesondere eine geradkettige Alkylgruppe mit 1 bis 20 C-Atomen, in dem auch ein oder mehrere H Atome durch F ersetzt sein können.

In einer bevorzugten Ausführungsform steht R¹ für N(R²)₂, eine geradkettige Alkylgruppe mit 1 bis 10 C-Atomen oder eine verzweigte oder cyclische Alkylgruppe mit 3 bis 10 C-Atomen, wobei die Alkylgruppe jeweils mit einem oder mehreren Resten R² substituiert sein kann und wobei eine oder mehrere nicht benachbarte CH₂-Gruppen durch O, NR², S, C=O, C(=O)O oder C(=O)NR² ersetzt sein können, oder eine Arylgruppe oder Heteroarylgruppe, die jeweils mit einem oder mehreren Resten R² substituiert sein können.

R² ist bei jedem Auftreten gleich oder verschieden H, D, F, Cl, Br, I, N(R³)₂, CN, NO₂, OR³, SR³, C(=O)OR³, C(=O)N(R³)₂, C(=O)R³, eine geradkettige Alkylgruppe mit 1 bis 10 C Atomen oder eine Alkenyl- oder Alkinylgruppe mit 2 bis 10 C-Atomen oder eine verzweigte oder cyclische Alkylgruppe mit 3 bis 10 C-Atomen, wobei die Alkyl-, Alkenyl- oder Alkinylgruppe jeweils mit einem oder mehreren Resten R³ substituiert sein kann, wobei eine oder mehrere nicht benachbarte CH₂-Gruppen durch R³C=CR³, C=C, C=O, NR³, O, S, C(=O)O oder C(=O)NR³ ersetzt sein können, oder eine Arylgruppe oder Heteroarylgruppe, die jeweils mit einem oder mehreren Resten R³ substituiert sein kann.

R³ ist bei jedem Auftreten gleich oder verschieden H, D, F OH oder eine geradkettige Alkylgruppe mit 1 bis 5 C-Atomen, in der auch ein oder mehrere H Atome durch F oder OH ersetzt sein können.

In einer besonders bevorzugten Ausführungsform steht R¹ für N(R²)₂, eine geradkettige Alkylgruppe mit 1 bis 6 C-Atomen oder eine verzweigte oder cyclische Alkylgruppe mit 3 bis 6 C-Atomen, wobei die Alkylgruppe jeweils mit einem oder mehreren Resten R² substituiert sein kann und wobei eine oder mehrere nicht benachbarte CH₂-Gruppen durch O, NR², S, C=O, C(=O)O oder C(=O)NR² ersetzt sein können, oder eine Arylgruppe oder Heteroarylgruppe mit 5 bis 10 aromatischen Ringatomen, die jeweils mit einem oder mehreren Resten R² substituiert sein kann.

R² ist bei jedem Auftreten gleich oder verschieden H, D, F, OH, C(=O)OH, eine geradkettige Alkylgruppe mit 1 bis 5 C-Atomen oder eine Arylgruppe oder Heteroarylgruppe mit 5 bis 10 aromatischen Ringatomen, in der auch ein oder mehrere an Kohlenstoff gebundene H Atome durch F oder NO₂ ersetzt sein können.

Besonders bevorzugt steht R¹ für eine substituierte oder unsubstituierte Methylgruppe, Ethylgruppe, Propylgruppe, bevorzugt substiuiert mit F oder COOH, oder für N(R²)₂, insbesondere für NHR², wobei R² für eine Arylgruppe oder Heteroarylgruppe mit 5 bis 10 Ringatomen steht, in der auch ein oder mehrere an Kohlenstoff gebundene H Atome durch F, OH, NH₂ oder NO₂ ersetzt sein können. Insbesondere bevorzugt steht R¹ für Methyl, CH₂-COOH oder NH-Phenyl, wobei das N an die SO₂-Gruppe gebunden ist.

In einer bevorzugten Ausführungsform der Erfindung basiert mindestens ein Makromer auf Poly(meth)acrylate wie Po-ly(meth)acrylamide, Poly(meth)acrylsäure, PolyHPMA oder PolyHEMA, Polyethylenglykol (PEG), Polyvinylalkohol (PVA), Polyurethan (PU), Polyvinylpyrrolidon (PVP), Polyamide, Po-ly(amidoamine) (PAMAM), Polyester, wie Polylactide, Polyglycolsäure (PGA) oder Poly(lactid-co-glycolid) (PLGA), Polyanhydride, Poly(ortho)ester, Polyacetale, Poloxamere (Blockcopolymere aus Ethylenoxid (PEG) und Propylenoxid (PPG)) wie PEG-Co-PPG-Co-PEG), Poly-2-oxazoline, Polyphosphazene, Polyglycerin, Polyamine wie Polylysin oder Polyethylenimin (PEI), Polycarbonate, Polyglutaminsäure, insbesondere Poly-Gamma-Glutaminsäure, Polyasparaginsäure (PASA), Polyphosphonate, und das andere Makromer auf DNA, RNA, Gelatine, Polyhydroxyalkanoate (PHA), Poly-Gamma-Glutaminsäure, Poly-Gamma-Glutaminsäure, Peptide wie Kollagene, VPM, Albumin oder Fibrin, Polysacccharide wie Agarose, Chitin, Chitosan, Chondroitin, Mannan, Inulin, Dextran, Cellulose, Alginate oder Hyaluronsäure. Dadurch ist es möglich in das Hydrogel eine biochemische Reaktivität zu integrieren, beispielsweise eine Spaltung oder Abbaufähigkeit, wie beispielsweise durch Estergruppen oder Carbonatgruppen im Makromer oder durch enzymatische Reaktionen. Beispiele für geeignete Peptide sind beispielsweise enzymatisch spaltbare Dithiolpeptide wie VPM (Sequenz: GCRDVPMSMRGGDRCG).

Bevorzugt werden beide Makromere so eingesetzt, dass die Anzahl der funktionellen Gruppen SH:Ar-SO₂-R¹ der beiden Makromere, die zur Vernetzung beitragen, bei 2:1 bis 1:2 liegt, bevorzugt bei 1,5:1 bis 1:1,5, besonders bevorzugt 1,2:1 bis 1:1,2, insbesondere bei 1:1. Werden mehrere unterschiedliche Verbindungen mit der jeweiligen funktionellen Gruppe eingesetzt, so beziehen sich die Angaben auf die gesamte Anzahl dieser Gruppen, beispielsweise bei Verwendung von unterschiedlichen Verbindungen mit Thiolgruppen. So kann beispielsweise eine Thiolverbindung zur Modifikation und eine andere Verbindung zur Vernetzung verwendet werden.

Bevorzugt liegen beide Makromere in Lösung vor, bevorzugt in wässriger Lösung. Es kann erforderlich sein, den pH-Wert anzupassen, bevorzugt durch Verwendung eines Puffers.

In einer bevorzugten Ausführungsform wird eine erste Lösung mit dem ersten Makromer umfassend Thiolgruppen und eine zweite Lösung mit dem zweiten Makromer umfassend die aromatische Sulfonylgruppe bereitgestellt. Diese beiden Lösungen werden dann miteinander kombiniert.

In einer bevorzugten Ausführungsform liegt der pH-Wert der verwendeten Lösungen der Makromere, insbesondere der Zusammensetzung, bei 6 bis 9 (bei 25 °C). Bevorzugt wird der pH-Wert durch einen Puffer eingestellt, bevorzugt mit einer Pufferkonzentration zwischen 5 mM bis 100 mM. Beispiele für Puffer sind PBS oder HEPES. Durch eine höhere Pufferkonzentration kann der pH-Wert im Gel bei Verwendung von hohen Makromerkonzentrationen stabilisert werden, da die Abgangsgruppe als Säure wirken kann. Bevorzugt ist ein pH-Wert von 6 bis 9, bevorzugt 6,5 bis 8, besonders bevorzugt 6,6 bis 7,5. Dadurch ist es möglich die Gelierungszeit zwischen beispielsweise 3 Sekunden (pH 8) bis 3,5 Minuten (pH 6,6) einzustellen (Gemessen bei 25 °C bei konstanter Makromerkonzentration).

Es ist auch möglich die Zusammensetzung bei einem ersten pH-Wert zu konstituieren, um dann die Vernetzungsreaktion durch Änderung des pH-Werts auf einen zweiten pH-Wert zu starten. Bevorzugt ist die Vernetzungsreaktion bei dem ersten pH-Wert mindestens stark verlangsamt, so liegt der erste pH-Wert bevorzugt außerhalb der vorstehend genannten Bereiche. Der zweite pH-Wert liegt bevorzugt innerhalb einem der vorstehend genannten Bereiche. Die pH-Änderung kann auch dadurch erreicht werden, dass die Zusammensetzung in ein Milieu mit entsprechendem pH-Wert gegeben wird. Bevorzugt liegt der zweite pH-Wert zwischen 6 bis 9, bevorzugt 6,5 bis 8, besonders bevorzugt 6,6 bis 7,5.

Außerdem kann die Reaktion durch Veränderung des pH-Wert gestartet, bzw. beschleunigt werden.

In einer weiteren bevorzugten Ausführungsform liegt der Gehalt an Makromer in der Zusammensetzung bei 1 bis 30 Gew.%, bevorzugt bei 3 bis 15 Gew.%, besonders bevorzugt bei 3 bis 10 Gew.%, bezogen auf alle eingesetzten Makromere.

Die Temperatur bei der Bildung des Hydrogels liegt bevorzugt zwischen 20 °C und 45 °C, bevorzugt zwischen 20 °C und 40 °C.

Die hier beschriebene Reaktion zur Bildung der Hydrogele zeichnet sich durch mehrere Vorteile aus. Im Unterschied zu bekannten Vernetzungsreaktionen ist sie unter physiologischen Bedingungen weder besonders schnell, noch besonders langsam, sie ist vielmehr unter anderem über den pH-Wert steuerbar. Dies ermöglicht die Einkapselung von Zellen oder anderen Stoffen wie Peptiden, Enzymen, chemischen Verbindungen oder ähnliches, während der Bildung des Gels. Die Zusammensetzung bleibt während der Bildung des Gels noch länger viskos, so dass sie noch länger mit geringen Scherkräften durchmischt werden kann. Dies ermöglicht eine homogene Verteilung der Zellen im Hydrogel ohne, dass weitere Schritte, wie Wenden des Gels während der Aushärtung, erforderlich sind.

Die vorgeschlagene Reaktion ist außerdem bei physiologischen Bedingungen ausreichend schnell. Dies ermöglicht die Verwendung in Zellkulturen, bevorzugt in dreidimensionaler Zellkultur oder sogar in situ. Auch kann die Gelierung über den pH-Wert gesteuert werden, was die Verwendung zum Aufbau von Gelen in situ, beispielsweise im 3D-Druck oder in einem Organismus ermöglicht wenn eine entsprechende Zusammensetzung injiziert wird.

In einer bevorzugten Ausführungsform werden die Bedingungen so gewählt, dass eine Gelierung innerhalb von 3 Sekunden bis 5 Minuten erreicht wird. Die Gelierungszeit kann dabei bevorzugt über die Makromerkonzentration, pH-Wert und Temperatur eingestellt werden. Dies erlaubt außerdem die Einstellung der physikalischen Eigenschaften der Gele, wie Langzeitstabilität, Quellverhalten und mechanische Eigenschaften.

Die Reaktion ist außerdem orthogonal zu OH-Gruppen, Aminogruppen, Carbonsäuregruppen und Acrylatgruppen, welche unter physiologischen Bedingungen nicht reagieren.

In einer bevorzugten Ausführungsform der Erfindung trägt die Reaktion der beiden Makromere ausschließlich zur Bildung des Hydrogels bei. Es finden keine anderen Vernetzungsreaktionen statt.

Durch die Wahl der aromatischen oder heteroaromatischen Gruppe, welche die Sulfonylgruppe trägt, und des pH-Werts kann die Geschwindigkeit der Reaktion gesteuert werden. So kann die Geschwindigkeit der Gelierung an die jeweilige Verwendung angepasst werden. Im Unterschied zu anderen Reaktionen muss kein Starter oder Beschleuniger hinzugegeben werden.

Das Verhältnis der beiden Makromere ist bevorzugt so gewählt, dass nach der Reaktion alle funktionellen Gruppen reagiert haben. Es kann davon abhängen, ob noch weitere Funktionalisierungen durchgeführt werden.

So ist es beispielsweise möglich, durch vorherige Zugabe von thiolhaltigen Verbindungen das zweite Makromer zu modifizieren, ehe die Vernetzung und Bildung des Hydrogels durch Zugabe des ersten Makromers eingeleitet wird. Dadurch kann das Hydrogel mit weiteren Funktionen modifiziert werden. Möglich sind beispielsweise Fluorophore oder bioaktive Reagenzien.

Beispiele für bioaktive Reagenzien sind Gewebewachstumspromotoren, Chemotherapeutika, Proteine (Glykoproteine, Collagen, Lipoproteine), Zellbindungsmediatoren, zum Beispiel Fibronectin, Laminin, Collagen, Fibrin, oder integrinbindende Sequenzen (zum Beispiel cyclo(RGDfC) oder cadherinbindende Sequenzen, Wachstumsfaktoren, Differizierungsfaktoren oder Fragmente der vorgenannten Reagenzien. Beispiele sind epidermaler Wachstumsfaktor EGF, endothelialer Wachstumsfaktor VEGF, Fibroblastenwachstumtsfaktoren wie bFGF, Insulinähnliche Wachstumgsfaktoren (z. B. IGF-I, IGF-II), transformierende Wachstumsfaktoren (z. B. TGF-α, TGF-β), DNA-Fragmente, RNA-Fragmente, Aptamere oder peptidomimetica, bevorzugt sind Zellbindungsmediatoren wie VEGF.

Die Modifikation kann beispielsweise dazu genutzt werden, um abhängig von den zu kultivierenden Zellen entsprechende Umgebungen im Hydrogel zu schaffen.

Die Reagenzien werden bevorzugt in wirksamen Konzentrationen eingesetzt, dies kann beispielsweise im Bereich von 0,01 bis 100 mM sein, bevorzugt 0,1 mM bis 50 mM, insbesondere 0,2 mM bis 10 mM, insbesondere 0,5 bis 5 mM bezogen auf das gequollene Gel.

Die Erfindung betrifft außerdem eine Zusammensetzung zur Herstellung eines Hydrogels umfassend mindestens zwei Makromere a1) und a2) wie für das Verfahren beschrieben.

Die Erfindung betrifft außerdem ein Hydrogel erhalten mit dem erfindungsgemäßen Verfahren.

Durch das erfindungsgemäße Verfahren erhält man ein Hydrogel umfassend eine erste Vielzahl von Makromeren, welche mit einer zweiten Vielzahl von Makromeren vernetzt ist, wobei die Vernetzung über eine Vielzahl von Ar-S-Bindungen erfolgt, wobei Ar eine aromatische oder heteroaromatische Gruppe ist.

Eine solche Bindung wird aus der nukleophilen Substitution durch Thiole an elektronenarmen Aromaten erhalten. Vorteilhafte Ausführungsformen werden für das Verfahren beschrieben.

Die erfindungsgemäßen Hydrogele sind lange Zeit stabil, bevorzugt bis zu 6 Wochen. Sie können auf einfache Weise und unter physiologischen Bedingungen modifiziert und erhalten werden. Sie eignen sich insbesondere zur Verkapselung von Zellen, für dreidimensionale Zellkulturen, Organoide, Biomaterialien, injizierbare Biomaterialien, Zelltherapien, Gewebemodifizierung, Geweberegeneration, Gewebetransplantation, regenerative Medizin, 3D Druck, 3D Bioprinting, Wundverbände oder Wundbehandlung, Transportmittel für Wirkstoffe, In-vitro-Modelle zum Untersuchen oder Testen von Diagnostika oder Therapeutika oder Zelltransplantationen.

Durch die Reaktion unter physiologischen Bedingungen ist die genannte Reaktion insbesondere im biologischen Bereich anwendbar. So ist es beispielsweise denkbar, dass die beiden miteinander reagierenden Makromere erst in situ miteinander kombiniert oder vermischt werden. Dies kann beispielsweise durch eine Mehrkomponentenspritze geschehen.

Die Erfindung betrifft ein Verfahren zur Umhüllung von Zellen, wobei das Hydrogel in Gegenwart der Zellen gebildet wird, um die Zellen zu umhüllen. Dies kann beispielsweise zur Zellkultur, insbesondere zur dreidimensionalen Zellkultur verwendet werden.

Die Erfindung betrifft außerdem ein Kit zur Herstellung eines Hydrogels umfassend die Makromere a1) und a2) wie für das Verfahren beschrieben.

Die beschriebene Reaktion eignet sich auch, um vorhandene Gele zusätzlich zu vernetzen. In einem solchen Verfahren wird ein Gel umfassend mindestens zwei der funktionalen Gruppen der Komponente a1) oder a2), wie beispielsweise aus A. Farrukh, J. I. Paez, M. Salierno, A. del Campo, Angew. Chem. Int. Ed. 2016, 55, 2092- 2096 durch Einpolymerisierung entsprechender Monomere in Polyacrylamidgele bekannt, bereitgestellt und mit einem Makromer mit entsprechenden funktionellen Gruppen gemäß dem Makromer a1) oder a2) umgesetzt, wobei in diesem Fall die Makromere a1) oder a2) mindestens zwei der funktionellen Gruppen aufweisen, so dass es zur Vernetzung des Gels durch diese Reaktion kommt.

Die Erfindung betrifft daher auch ein Verfahren zur Modifikation von Gelen, umfassend die Schritte:
a) Bereitstellen eines Gels, umfassend mindestens zwei funktionelle Gruppen gemäß Komponente a1) oder mindestens zwei funktionelle Gruppen gemäß Komponente a2);
b) Zugabe einer Zusammensetzung umfassend mindestens ein Makromer gemäß der jeweils anderen Komponente, wobei das Makromer mindestens zwei funktionelle Gruppen aufweist;
c) Modifikation des Gels davon durch Reaktion der funktionellen Gruppen, wobei die Reaktionsbedingungen so gewählt werden, dass die Thiolgruppen eine nukleophile aromatische Substitution an der Gruppe Ar durchführen können, wobei die Gruppe SO₂-R¹ als Abgangsgruppe dient.

Bevorzugt wird das Verfahren nach der Herstellung des Gels zur nachträglichen Modifikation verwendet. Dadurch ist es möglich, das Gel unter physiologischen Bedingungen zu modifizieren, um beispielsweise seine mechanischen Parameter anzupassen.

Durch die pH-Abhängigkeit und/oder Temperatur der Reaktion ist es beispielsweise möglich, dass diese Modifikation erst bei einer bestimmten Änderung der Bedingungen eintritt.

### Beispiele

Die Ausführungsbeispiele sind in den Figuren dargestellt. In den Beispielen werden die Makromere als Polymere bezeichnet.
- Fig. 1: Schematische Darstellung der Herstellung eines Hydrogels gemäß der Erfindung;
- Fig. 2: a) Foto eines PEG-Thiol-MS Hydrogels (5 Gew.% Polymerkonzentration in 10 mM HEPES Puffer); b) Schermodule während der Gelierung (5 Gew.% Polymer, 10 mM HEPES Puffer pH 6,6, T= 25°C);
- Fig. 3a: Schermodule während der Gelierung der verschiedenen Hydrogele bei 25 °C (jeweils 5 Gew.%; 10 mM HEPES Puffer; pH 8);
- Fig. 3b: Schermodule während der Gelierung der verschiedenen Hydrogele bei 37 °C (jeweils 5 Gew.%; 10 mM HEPES Puffer; pH 8);
- Fig. 4: Wirkung des pH-Wertes (bei 5 Gew.% Polymergehalt, 25°C) auf die Vernetzungskinetik und die Schermodule (a) Thiol-Mal, Thiol-MS, b) Thiol-VS);
- Fig. 5: Schermodul in Abhängigkeit von Temperatur (Bedingungen: bei 30 min, 5 Gew.%, pH 7,0);
- Fig. 6: Einfluss von Polymergehalt und HEPES-Pufferkonzentration auf die mechanischen Eigenschaften (Säulen, linke Skala) und den pH-Wert (Quadrate, rechte Skala) der hergestellten Thiol-MS-Gele. Bedingungen: pH = 7,5, T = 25°C, bei 60 min;
- Fig. 7: Vergleich der normierten Masse von geschwollenen Thiol-X-Gelen. Gele wurden über 6 Wochen, bzw. 4 Wochen lang im Zellkulturmedium bei 37°C inkubiert (a) 10 Gew.% Polymeranteil, pH 8,0; b) 5 Gew.% Polymergehalt bei pH 7,0). Unter diesen Bedingungen hergestellte Thiol-MS-Gele sind auch nach 6 Wochen Inkubation im Zellkulturmedium hydrolysestabil;
- Fig. 8: Fibroblast L929 verkapselt in den verschiedenen enzymatisch spaltbaren Thiol-X-Hydrogelen. Lebend / tot Assay von L929 Fibroblasten-Einzelzellen, die für 1 Tag in den Materialien (a-c) verkapselt waren: Im Vergleich zu den anderen Systemen zeigten Zellen, die in Thiol-MS-Hydrogelen kultiviert wurden, eine homogenere Verteilung über das gesamte Material (a, Z-Stapel) und eine ähnliche Lebensfähigkeit (c);
- Fig. 9: (a) Schemata von enzymatisch spaltbaren Gelen, die zur Verkapselung von Zellsphäroiden verwendet werden. (b-c) Migrationsverhalten von Zellen aus verkapselten Sphäroiden. Die Ergebnisse des Migrationstests nach 3-tägiger Kultur zeigten, dass die Migrationsstrecke in Thiol-MS-Gelen dazwischen lag. Färbung: FITC-Phalloidin (Aktinfasern), DAPI (Kern);
- Fig. 10: Morphologie einzelner Zellen (Mausfibroblast L929), die nach 3 Tagen Zellkultivierung in den verschiedenen enzymatisch spaltbaren Thiol-X-Hydrogelen eingeschlossen sind. Im Vergleich zu den anderen Systemen zeigten Zellen, die in Thiol-MS-Hydrogelen kultiviert wurden, eine homogenere Verteilung, weniger Clusterbildungen oder Aggregationen. Färbung: FITC-Phalloidin (Aktinfasern), DAPI (Kern); Der Maßstab ist jeweils 50 µm;

### Chemische Synthese

Chemikalien und Lösungsmittel wurden in p.a. Reinheit erworben und direkt eingesetzt, wenn nicht anders beschrieben. 4-(5-(Mehtylsulfonyl)-1,3,4-oxadiazol-2-yl)anilin wurde von Ark Pharm USA erworben. 4-armige Polyethyhenglykolpolymere (PEG, 20 kDa auf der Basis von Pentaerythrit) funktionalisiert mit Maleimid (PEG-Mal), Vinylsulfon (PEG-VS), Thiol (PEG-SH) und N-Succinimidylcarboxymethylester (NHS-PEG) und lineares methoxyliertes PEG Polmyer (5 kDa) ebenso jeweils funktionalisiert mit NHS, SH, Mal und VS wurden von Jenkem, USA erworben. Pufferlösungen wurden frisch hergestellt. 10 mM HEPES (pH 8,0, 7,0, und 6.7) und phosphatgepufferte Salzlösung (PBS, pH 7,4 und 7,0) wurden verwendet.

Deuterierte Lösungsmittel wurden von der Deutero GmbH Deutschland (D-56288 Kastellaun) bezogen. Deuterierte Phosphatpuffer-Salzlösung (PBS) wurde hergestellt, indem die richtige Menge an Dinatriumphosphat, Mononatriumphosphat, Natriumchlorid und Kaliumchlorid in D₂O gelöst wurde; gefolgt von einer pD-Einstellung mit 20 %iger DCl-Lösung (Merck), bis die pD-Werte von 8,0; 7,4; 7,0; 7,0; und 6,0 erreicht wurden. Der pH-Wert wurde mittels pH-Meter überwacht und der folgende Korrekturfaktor wurde angewendet: pD = pH_{obs} + 0,4. (siehe Bates et al Anal. Chemie. 1968 40 (4), 700-706).

Dünnschichtchromatographie (TLC)-Platten (ALUGRAM^{®} SIL G/UV254) und Kieselgel für die Säulenchromatographie (60Å Porengröße, 63-200µm Partikelgröße) wurden von Macherey-Nagel, (52355 Düren) Deutschland bezogen. TLC-Platten wurden unter 254 oder 365 nm Licht beobachtet. Die HPLC-Analyse und -Reinigung der Verbindungen wurde mit einer HPLC JASCO 4000 (Japan) durchgeführt, die mit einem Diodenarray, UV-Vis-Detektor und Fraktionssammler ausgestattet ist. Reprosil C18-Säulen wurden für semipräparative (250 × 25 mm) und analytische (250 × 5 mm) Durchläufe verwendet. Es wurden Lösungsmittelgradienten unter Verwendung einer Kombination der folgenden Eluenten verwendet: Lösungsmittel A (MilliQ-Wasser + 0,1% TFA) und Lösungsmittel B (95% ACN/5% MilliQ-Wasser + 0,1% TFA), typischerweise über 40 Minuten. Die Reinigung von modifizierten Polymeren erfolgte typischerweise durch Dialyse gegen Aceton und Wasser. Zum Einsatz kamen Spectra/Por 3 Dialyseschläuche (Molekulargewichtsausschlussgrenze MWCO= 3,5 kDa) von Spectrum Inc..

Die Lösungsspektren ¹H-NMR und ¹³C-NMR wurden bei 25 °C auf einer Bruker Avance 300 MHz oder auf einer Bruker Avance III UltraShield 500 MHz aufgenommen. Letzteres war mit einem Hegekühlten 5 mm TCI-CryoProbe, eine protonenoptimierte Dreifachresonanz-NMR-Inverse-Sonde mit externer Wasserkühlung (CP TCI 500S2, H-C/N-D-05 Z) ausgestattet. Sofern nicht anders angegeben, wurden alle Messungen bei 298K durchgeführt. Tetramethylsilan (TMS) (δ= 0 ppm) wurde als interne Referenz verwendet. Die chemischen Verschiebungen sind angegeben in Teile ppm und die Kopplungskonstanten in Hertz. Folgende Abkürzungen werden verwendet: s-singlet, d-doublet, t-triplet, q-quartet, m-multiplet. Der Substitutionsgrad des PEG-Polymers wurde berechnet durch Endgruppenbestimmung. Das Integral des Signals, das dem PEG-Backbone entspricht (3,70-3,40 ppm), wurde auf 440H eingestellt und mit dem Integral der Protonen verglichen, das dem eingearbeiteten Molekül 2 (das aromatische -CHs bei 8,10-7,70 ppm und das Methylen bei 4,20 ppm). Funktionalisierungsgrade von >91% und Renditen von >91% wurden in allen Fällen erreicht. Die Daten wurden in MestReNova analysiert.

Massenspektren wurden mit Agilent Technologies 1260 Infinity Liquid Chromatography/Mass aufgezeichnet. Selektiver Detektor (LC/MSD) und 6545 Accurate-Mass Quadrupol Laufzeit (LC/Q-TOF-MS) unter Verwendung der chemischen Ionisierung durch Elektrospray. UV/VIS-Spektren wurden mit einer Varian Cary 4000 UV/VIS aufgenommen. Spektrometer (Varian Inc. Palo Alto, USA).

Die rheologischen Eigenschaften von Hydrogelen wurden an einem Discovery HR-3 Rheometer (TA Instruments, USA), ausgestattet mit 12 mm parallelen Platten und Peltier-Tisch, bei 25 und 37°C. Software war Trios v4. Daten wurden in Origin 9.1 aufgezeichnet und analysiert.

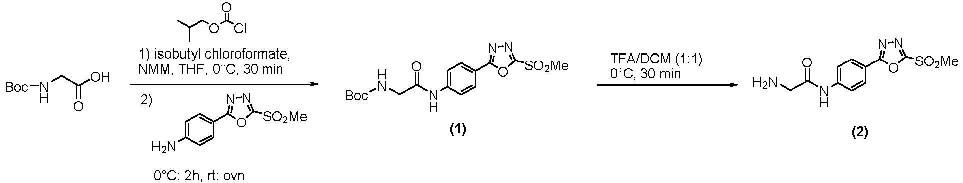

Die folgenden Protokolle wurden mit einigen Modifikationen übernommen: G. Liang et al Chem. Commun., 2017, 53, 3567-3570; J. Ling et al ChemBioChem 2018, 19, 1060.

### Synthese von tert-Butyl (2-((4-(5-(methylsulfonyl)-1,3,4-oxadiazol-2-yl)phenyl)amino)-2-oxoethyl)Carbamat (1):

Boc-Gly-OH (1 Äq., 2,28 mmol, 0,394 g) wurde in wasserfreiem THF (3 mL) bei 0 °C gelöst. Isobutylchlorameisensäureester (1,2 Äq., 2,85 mmol, 0,314 mL) und N-Methylmorpholin (2,6 Äq., 5,7 mmol, 0,627 mL) wurden der Lösung mit einer Spritze unter Stickstoffatmosphäre sorgfältig zugegeben und 30 Minuten lang gerührt. Eine Lösung von 4-(5-(Methylsulfonyl)-1,3,4-oxadiazol-2-yl)anilin (0,25 Äq., 0,57 mmol, 0,136 g) in THF (3 mL) wurde der Mischung tropfenweise zugegeben, für weitere 2 h bei 0°C und dann über Nacht bei Raumtemperatur gerührt. Gesättigtes NaHCO₃ wurde zugegeben und das Reaktionsgemisch mit Ethylacetat (2 x 30 mL) extrahiert. Die kombinierte, organische Phase wurde mit Natriumsulfat getrocknet, gefiltert, verdampft, durch präparative HPLC aufgereinigt (5B bis 95B 280 nm, Reaktionszeit = 28 min) und ein weißer Feststoff wurde nach der Gefriertrocknung erhalten, 165 mg (Ausbeute= 73%).

### Synthese von 2-Amino-N-(4-(5-(5-(Methylsulfonyl)-1,3,4-oxadiazol-2-yl)phenyl)acetamid (2):

Die Verbindung 1 (45 mg) wurde in 1:1 TFA/DCM (2 mL) gelöst, bei Raumtemperatur 2 h lang gerührt und unter Stickstofffluss verdampft. Das Endprodukt wurde nach der HPLC-Reinigung (5B bis 95B 280 nm, Reaktionszeit = 18 min) erhalten, (Y = >99%). Die reine Verbindung wurde sofort an das PEG-Polymer gekoppelt, da ansonsten eine Zersetzung innerhalb von 1 Woche nach der Lagerung bei -20°C beobachtet wurde.

### Synthese von PEG-MS:

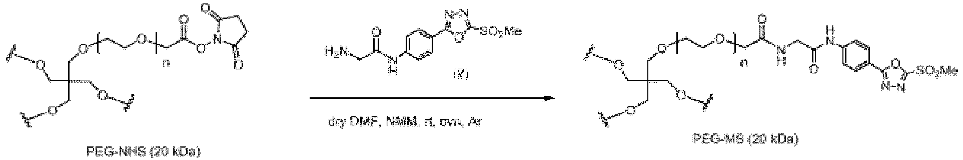

Die frisch zubereitete Verbindung 2 (50 µmol, 15 mg) und N-Methylmorpholin (18 µmol, 20 µL) wurden in trockenem DMF (2 mL) gelöst, mit Stickstoff gespült und für 15 min gerührt. 20 kDa, 4-armiges PEG-NHS (100 mg, 5 µmol) wurde in trockenem DMF (1 mL) gelöst und unter Stickstoffstrom zugegeben. Die Mischung wurde über Nacht bei Raumtemperatur unter inerter Atmosphäre gerührt, dann in Aceton und Wasser dialysiert und gefriergetrocknet. Ein weißes festes Polymer wurde erhalten und mittels ¹H-NMR in DCM-d₂ charakterisiert. Ein Funktionalisierungsgrad von >91% und eine Ausbeute von >90% wurden berechnet.

Die 2-(Methylsulfonyl)-5-phenyl-1,3,4-oxadiazolgruppe wurde als MS-Substrat für die Thiolkupplung ausgewählt. Unter den beschriebenen heteroaromatischen MS-Ringen reagiert dieses Substrat mit Thiolen unter hohem Umsatz und mittlerer Reaktionsgeschwindigkeit. [N. Toda, S. Asano, C. F. Barbas, Angew. Chem., Int. Ed. 2013, 52, 12592-12596. X. Chen, H. Wu, C.-M. Park, T. H. Poole, G. Keceli, N. O. Devarie-Baez, A. W. Tsang, W. T. Lowther, L. B. Poole, S. B. King, M. Xian, C. M. Furdui, ACS Chemical Biology 2017, 12, 2201-2208.] 4-armige PEG-MS-Makromere (20 kDa) wurden im 500-mg Maßstab in guter Ausbeute (Substitutionsgrad >91%) innerhalb von drei Syntheseschritten synthetisiert.

### Rheologische Messungen an Hydrogelen

Die Gelierung des 4-Arm-PEG-MS und 4-Arm-PEG-Thiol-Gemisch wurde untersucht. Angewandte Vernetzungsbedingungen betrugen 5

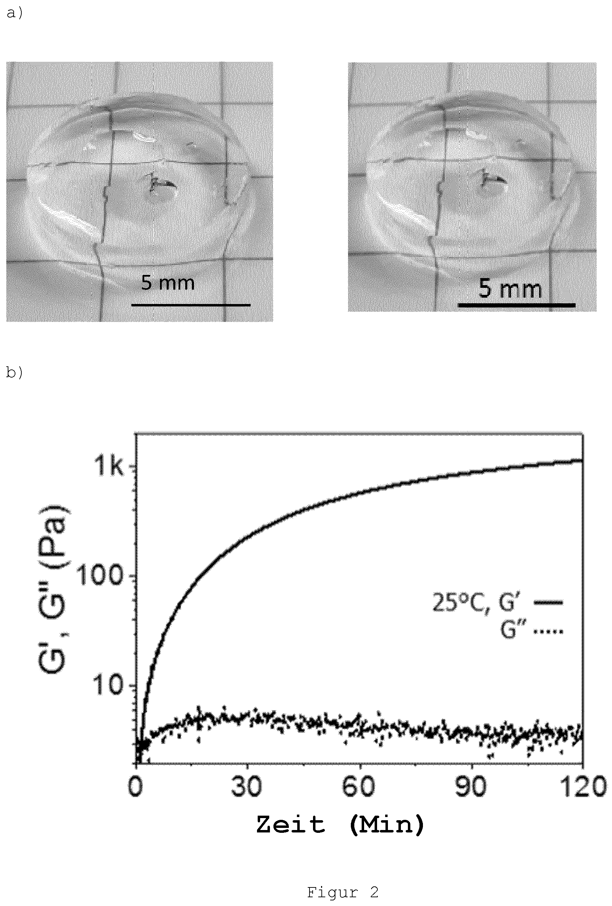

Gew.% Polymergehalt in 10 mM HEPES-Puffer, pH 6,6, bei 25°C. Ein MS:Thiol-Verhältnis von 1:1 wurde für die Experimente verwendet. Studien zeigten, dass Thiol-MS-Gele ein vernetztes Gel innerhalb von 3-4 min bildeten (siehe Tabelle 1). Dies entspricht einer günstigen Vernetzungszeit, die ein einwandfreies Mischen und eine Homogenisierung von Vorläuferlösungen ermöglicht. Rheologische Untersuchungen ergaben, dass die vernetzten Gele ein Scherspeichermodul von G'~1 kPa erreichen (Figur 2b). Ein gequollenes PEG-Thiol-MS-Hydrogels (5 Gew.% Polymerkonzentration in 10 mM HEPES-Puffer zeigt Figur 2a.

20 kDa, 4-armige PEG-X Polymerlösungen wurden frisch hergestellt und für diese Studien verwendet. Das Polymer wurde in dem entsprechenden Lösungsmittel gelöst, mithilfe eines Vortexers gemischt, in ein Ultraschallbad gehalten und zentrifugiert, um Blasen zu entfernen. 21 µL einer 5% w/v PEG-X-Lösung wurde auf die Peltier-Unterplatte des Rheometers aufgetragen, gefolgt von 21 µL einer 5% w/v PEG-Thiol-Lösung und dem Mischen mit der Pipettenspitze direkt auf der Platte. Die obere Platte wurde angenähert, um die Probe zwischen die beiden Platten zu legen, und anschließend wurde die Probe mit Paraffinöl versiegelt, um eine Verdampfung während der Messung zu vermeiden. Die Gesamtzeit für die Probenbeladung mitsamt dem Start der Messung betrug ca. 2-3 min.

Die Gelierzeit und der Endschermodul des Hydrogels wurden rheometrisch bestimmt. Dehnungsdurchläufe (0,1 bis 1000% Dehnung bei Frequenz = 1 Hz) und Frequenzdurchläufe (0,01 bis 100 Hz bei Dehnung = 1%) wurden durchgeführt, um das lineare, viskoelastische Regime zu bestimmen. Die Zeitdurchläufe wurden innerhalb des linearen viskoelastischen Regimes mit folgenden Parametern durchgeführt: Spalt 300 µm, Axialkraft (0,0±0,1 N), Frequenz 1Hz, Dehnung 1%, Temperatur = 25 oder 37°C.

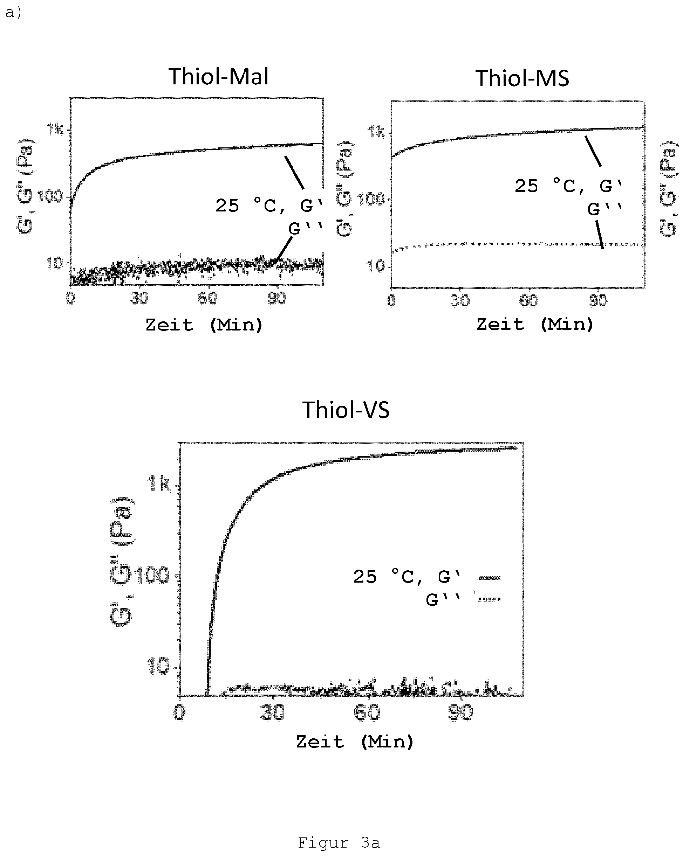

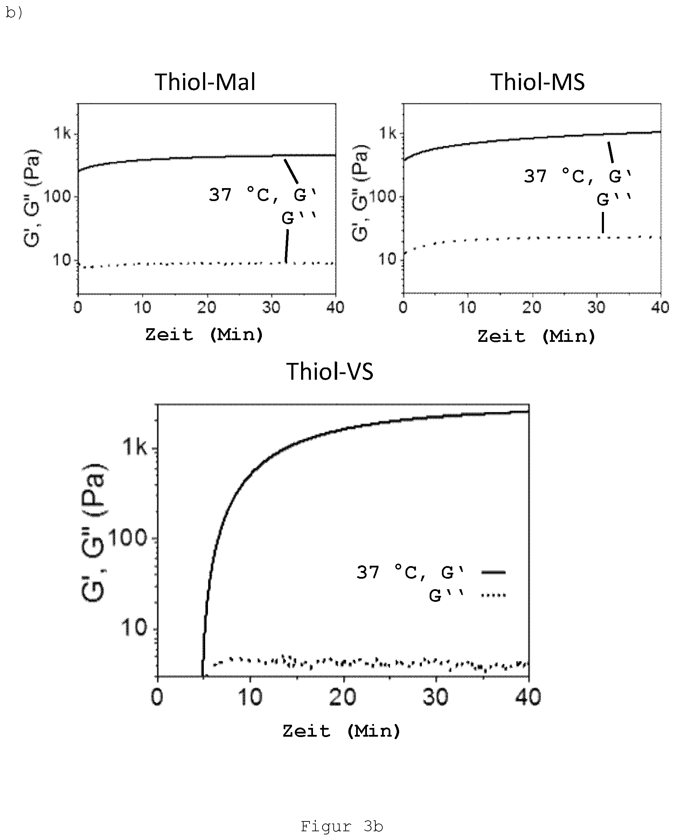

Hydrogele wurden bei 5 Gew.% Polymergehalt, HEPES-Puffer pH 8,0 und T = 25°C (Figur 3a) und T- 37 °C (Figur 3b) hergestellt.

Figur 3a vergleicht die Vernetzungskinetik von Thiol-MS mit der von Thiol-Mal- und Thiol-VS-Systemen. Die Experimente wurden unter zellkulturtypischen Bedingungen durchgeführt (5 Gew.% Polymer, in 10 mM HEPES-Puffer pH 8,0, bei 25°C). [E. A. Phelps, N. O. Enemchukwu, V. F. Fiore, J. C. Sy, N. Murthy, T. A. Sulchek, T. H. Barker, A. J. Garcia, Advanced Materials 2012, 24, 64-70. A. Farrukh, J. I. Paez, A. del Campo, Advanced Functional Materials 2019, 29, 1807734.] Unter diesen Bedingungen bildete sich das Thiol-MS-Gel in 3-4 s (Tabelle 1). Dies entspricht einer kurzen Vernetzungszeit, ist aber akzeptabel zum Mischen und Homogenisieren von Gelvorläufern. Im Vergleich dazu benötigte das Thiol-Mal-Gel 1 s zur Vernetzung und es wurden inhomogene Gele erhalten, während das Thiol-VS-System eine Gelierungszeit von ca. 10 min aufwies und ca. 2 h brauchte, um die Vernetzung zu vervollständigen. Diese Ergebnisse zeigen den folgenden Trend in der Gelierungsrate: Thiol-Mal > Thiol-MS > Thiol-VS, in Übereinstimmung mit den angegebenen Reaktionsraten für Modellverbindungen, [X. Chen, H. Wu, C.-M. Park, T. H. Poole, G. Keceli, N. O. Devarie-Baez, A. W. Tsang, W. T. Lowther, L. B. Poole, S. B. King, M. Xian, C. M. Furdui, ACS Chemical Biology 2017, 12, 2201-2208. F. Saito, H. Noda, J. W. Bode, ACS Chemical Biology 2015, 10, 1026-1033. H. Wang, F. Cheng, M. Li, W. Peng, J. Qu, Langmuir 2015, 31, 3413-3421.] wie in Tabelle 2 gezeigt.

Die Werte des Schermoduls der vernetzten Gele nach 1 h betrugen jeweils G'_{25°C} = 2000 Pa für Thiol-VS, 1000 Pa für Thiol-MS und 470 Pa für Thiol-Mal. Die höhere Steifigkeit von Thiol-MS-Gelen ist wahrscheinlich auf eine höhere Homogenität des Systems als Folge der langsameren Gelierungskinetik zurückzuführen, was zu weniger Netzwerkdefekten und zu einem höheren Vernetzungsgrad führt.

Dieses Ergebnis widerspricht früheren Reaktivitätsstudien von Thiol-Mal- und Thiol-MS-Kupplungen an kleinen Modellmolekülen, bei denen ähnliche Reaktionsumsätze in Phosphatpuffer-Salzlösung (PBS) pH 7,4 [N. Toda, S. Asano, C. F. Barbas, Angew. Chem., Int. Ed. 2013, 52, 12592- 12596.] gezeigt wurden. Wir nahmen an, dass die Hydrolyse von Mal-Gruppen, die bei basischem pH auftritt, der Grund für die geringeren mechanischen Eigenschaften von Thiol-Mal sein könnte. Um diese Hypothese zu überprüfen, wurde die Stabilität einer 4 Gew.% PEG-Mal-Lösung in deuteriertem PBS bei pD 8,0 durch ¹H-NMR untersucht.

Die Hydrolyse von Mal-Gruppen wurde nach 2 Stunden nachgewiesen. Es wird daher nicht erwartet, dass die Hydrolyse von Mal-Gruppen die mechanischen Eigenschaften von Thiol-Mal innerhalb des Bereichs der getesteten Bedingungen signifikant beeinflusst. Thiol-VS erreichte den höchsten Schermodul; Dies hängt mit einer höheren Umwandlung oder der langsamsten Aushärtung zusammen, die ein Netzwerk mit weitaus weniger Fehlern gewährleisten. Insgesamt zeigen diese Ergebnisse, dass die Thiol-MS-Vernetzung eine Zwischenkinetik zwischen dem sehr schnell vernetzenden Thiol-Mal und den langsamen Thiol-VS-basierten Materialien darstellt. Die beobachtete Vernetzungszeit im Bereich von wenigen Sekunden ermöglicht ein komfortables Mischen und Pipettieren der Komponenten bei geringen Scherkräften und dürfte für die Zellverkapselung geeignet sein.

### Abhängigkeit vom pH-Wert

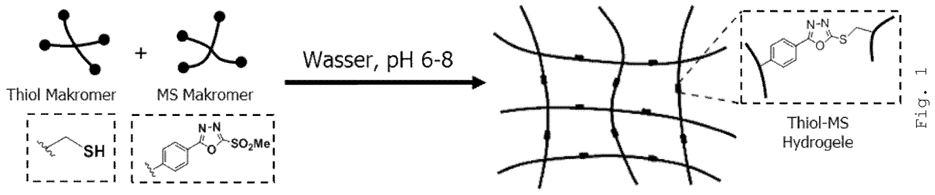

Die Gelierung des 4-Arm-PEG-MS und 4-Arm-PEG-Thiol-Gemisch wurde untersucht (Figur 1). Angewandte Vernetzungsbedingungen betrugen 5 Gew.% Polymergehalt in 10 mM HEPES-Puffer, pH 6,6, bei 25°C. Ein MS:Thiol-Verhältnis von 1:1 wurde für die Experimente verwendet. Die Gelierungszeit im Bulk für die Thiol-X-Hydrogele wurde bei unterschiedlichen pH-Werten bestimmt. Die Experimente wurden bei 5 Gew.% Polymerlösung, in 10 mM HEPES-Puffer, T = 25°C durchgeführt. Die Gelierzeit wurde als Zeitspanne zwischen dem Mischen der Komponenten und dem Zeitpunkt geschätzt, bei dem das Pipettieren zu der Mischung nicht mehr möglich war. Studien zeigten, dass Thiol-MS-Gele ein vernetztes Gel innerhalb von 3-4 min bildeten (siehe Tabelle 1). Dies entspricht einer günstigen Vernetzungszeit, die ein einwandfreies Mischen und eine Homogenisierung von Vorläuferlösungen ermöglicht.

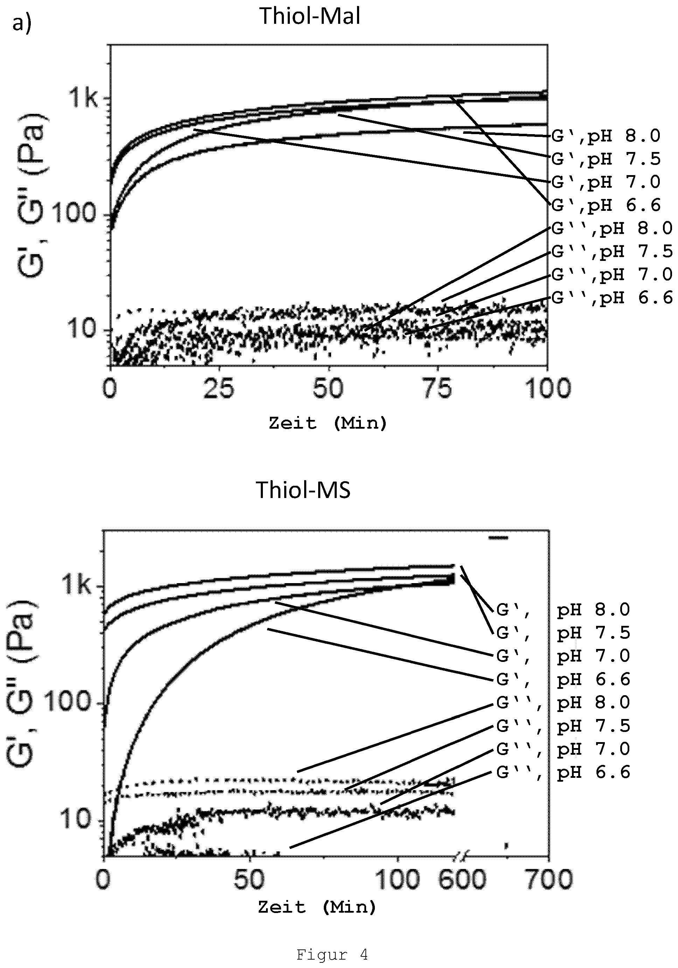

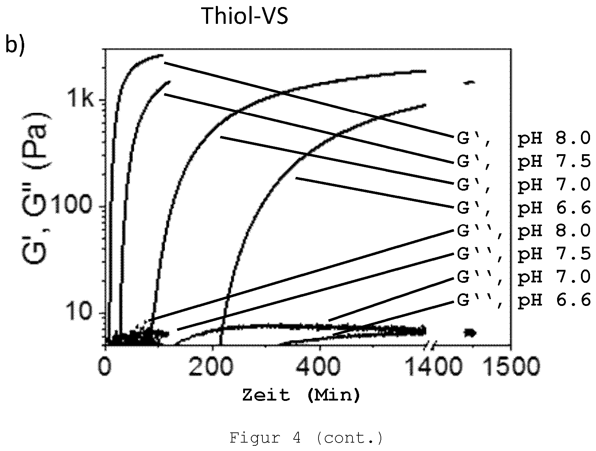

Die Reaktionsgeschwindigkeit der polaren Thiol-X-Kopplung ist abhängig vom pH-Wert im pH-Bereich zwischen 6 und 9. Dies ist auf die Deprotonierung der Thiolgruppe (pKa ~ 8) zum Thiolatanion zurückzuführen, das bei diesen Reaktionen als Nukleophil wirkt [M. H. Stenzel, ACS Macro Letters 2013, 2, 14-18.] Dieses Merkmal bietet eine interessante Möglichkeit für pH-gesteuerte Härtungskinetiken unter physiologisch relevanten Bedingungen. Es wurde die Thiol-MS-Vernetzung im pH-Bereich von 8,0-6,6 analysiert. Eine Abnahme der Vernetzungsrate wurde bei sinkendem pH-Wert beobachtet (Figuren 4a und 4b und Tabelle 1). Bemerkenswert ist, dass die pH-Änderung von 8,0 auf 6,6 eine Abstimmung der Gelierzeit von einigen Sekunden auf ein paar Minuten ermöglichte (Tabelle 1), was ein ideales experimentelles Zeitfenster für 3D-Zellverkapselungsanwendungen bietet. Im Gegensatz dazu variierte die Gelierzeit von Thiol-Mal nur innerhalb weniger Sekunden, während Thiol-VS zwischen einigen Minuten und einigen Stunden lag. Diese Ergebnisse verdeutlichen die Vorteile von Thiol-MS-Gelen in Bezug auf Handhabung und Anpassungsfähigkeit an die Anwendungsanforderungen gegenüber Thiol-Mal und Thiol-VS.

Der Schermodul von Thiol-MS-vernetzten Hydrogelen wurde durch den pH-Wert leicht beeinflusst: Bei pH 8,0 zeigten sie einen niedrigeren G', wahrscheinlich aufgrund des Auftretens der sehr schnellen Vernetzung, die zu einigen Inhomogenitäten und Defekten im Netzwerk führte. Dies war nicht der Fall bei Thiol-MS-Gelen, die bei pH 7,5-6,6 gebildet wurden, in denen ähnliche endgültige G's erhalten wurden. Daher scheint dies das optimale Intervall zu sein, in dem die Vernetzungsrate eingestellt werden kann, ohne die Qualität und mechanische Stabilität des Gels zu beeinträchtigen. Im Vergleich dazu zeigte das Thiol-Mal-System einen Rückgang der mechanischen Eigenschaften bei pH ≥ 7,5, aber einen ähnlichen endgültigen Schermodul bei pH = 7,0-6,6, während Thiol-VS einen klaren Trend zu einer langsameren Gelierungskinetik und einen leicht verringerten Schermodul bei abnehmendem pH-Wert zeigte. Dazu wurden Messungen in 10 mM HEPES-Puffer bei pH-Werten von 8,0; 7,5; 7,0 und 6, 6 bei 5 Gew.% Polymergehalt und bei 25 °C durchgeführt. Dabei ist zu beachten, dass die am schnellsten aushärtenden Systeme (Mal bei pH ≥ 7.0 und MS bei pH ≥ 7.5) unter Beladung des Rheometers sofort aushärten. (Figuren 4a und 4b)

### Einfluss der Temperatur auf die Gelierzeit

20 kDa, 4-armige PEG-X Polymerlösungen wurden frisch hergestellt und für diese Studien verwendet. Das Polymer wurde in dem entsprechenden Lösungsmittel gelöst, mithilfe eines Vortexers gemischt, in ein Ultraschallbad gehalten und zentrifugiert, um Blasen zu entfernen. 30 µL einer 5%-igen w/v PEG-X-Lösung wurde in ein Kunststoff-Eppendorf-Röhrchen gegeben, gefolgt von 30 µL einer 5%-igen w/v PEG-Thiol-Lösung unter kontinuierlichem Mischen mit der Pipette. Die Gelierzeit "im Bulk" wurde als die Zeit registriert, in der die Härtemischung nicht mehr länger floss und ein kontinuierliches Pipettieren nicht möglich war. Die Temperatur wurde mit einem Wasserbad mit Temperaturregelung kontrolliert.

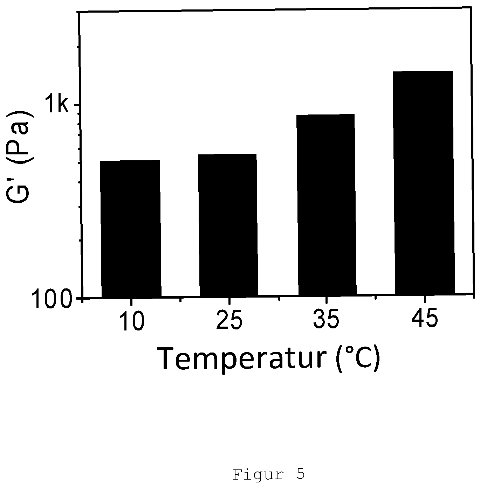

Die Temperatur kann auch zur Einstellung der Thiol-MS-Geleigenschaften verwendet werden. Die Temperaturabsenkung im Bereich von 45°C bis 25°C ermöglichte eine Abnahme des Schermoduls (Figur 5) und eine Verlängerung der Gelierzeit (siehe Tabelle 3).

### Einfluss von Polymergehalt und HEPES-Pufferkonzentration auf Thiol-MS-Hydrogele

Es wurden Hydrogele mit steigenden Polymergehaltswerten von 1,3; 2,5; 5,0; 7,5; und 10,0 Gew.%; bei konstantem pH = 7,5 und T = 25°C, entweder mit 10 mM oder 50 mM HEPES-Pufferkonzentration hergestellt. Der pH-Wert der präparierten Hydrogele wurde mit einem pH-Meter mit ebener Oberflächenelektrode (PH100 Waterproof ExStik^{®}, Extech Instruments, USA) gemessen.

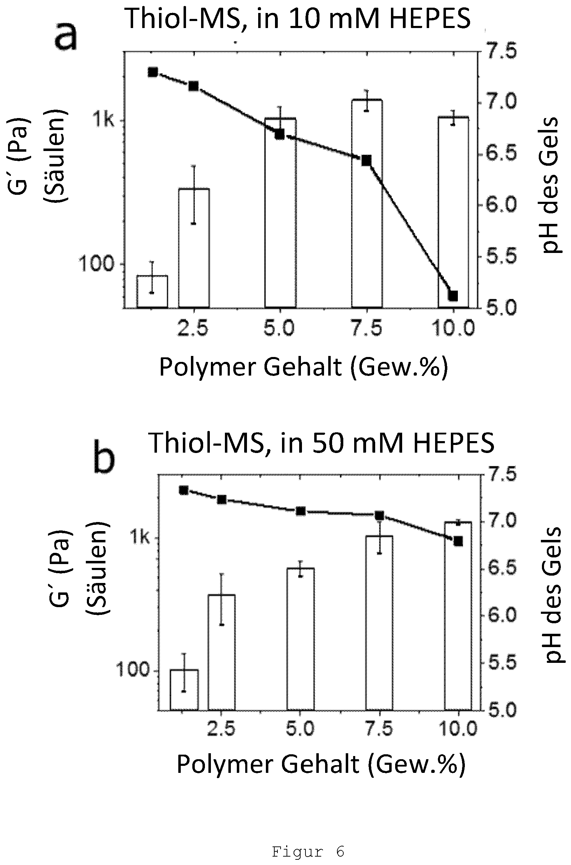

Schließlich wurde der Einfluss des Polymergehalts auf die Thiol-MS-Hydrogel-Vernetzungskinetik und den Schermodul untersucht (Figur 6a, 6b). Die Gelierzeit bei steigendem Polymergehalt war kürzer (im Bereich von 18-2 s, siehe Tabelle 4). Darüber hinaus stieg **G'** mit einem Anstieg der Polymerkonzentration von 1,3 auf 7,5 Gew.% und sank bei Polymerkonzentrationen über 10 Gew.%. Dieses Ergebnis war überraschend, da bereits bei 10 Gew.% Vorläuferlösungen richtig homogenisiert werden konnten; daher wurde nicht erwartet, dass ein schlechter Vermischungseffekt für dieses Verhalten verantwortlich ist.

Zur Untersuchung des Reaktionsmechanismus wurde der pH-Wert der resultierenden Gele gemessen (siehe Figur 6a, 6b). Es stellte sich heraus, dass der pH-Wert von frisch hergestellten Thiol-MS-Gelen mit zunehmender Polymerkonzentration abnahm. Gele zwischen 1,3-7,5 Gew.% zeigten einen pH-Wert zwischen 7,5-6,5, während Gele bei 10 Gew.% einen pH-Wert in der Nähe von 5,1 hatten. Dies lässt sich durch die Freisetzung einer Methansulfinsäure als Abgangsgruppe bei der Thiol-MS-Kopplung erklären. Bei hohem Polymeranteil wird die Abgangsgruppe in größeren Konzentrationen produziert, was zu einer pH-Wert-Absenkung des Vernetzungsmediums und damit zu einer Abnahme des erreichten Endschermoduls führt. Dieser Effekt kann durch Erhöhung der Pufferkapazität des Vernetzungsmediums gesteuert werden, was durch Erhöhung der HEPES-Pufferkonzentration von 10 mM auf 50 mM erreicht wird (siehe Figur 6). Letzteres ist bekanntlich noch zytokompatibel. Diese Ergebnisse zeigen, dass der Gehalt an Polymer auch dazu verwendet werden kann, die mechanischen Eigenschaften des Gels zu steuern. Bei höherer Konzentration (10 Gew.%), sollte der pH-Wert durch Erhöhung der Pufferkapazität kontrolliert werden.

### Quellungsmessungen an Thiol-X-Hydrogelen

Für diese Studien wurden Vorläuferlösungen von 5% w/v verwendet, welche in 10 mM HEPES-Puffer pH 7,0 hergestellt, und zuvor im Eisbad gekühlt wurden. 50 µL einer 5% w/v PEG-X-Lösung wurde in eine flexible PDMS-Zylinderform (0,75 cm Durchmesser) gegeben, schnell mit 50 µL einer 5% w/v PEG-Thiol-Lösung gemischt und in einer feuchten Kammer bei 37°C für 4h vernetzt. Die resultierenden Hydrogele wurden sorgfältig entfernt, 24 Stunden lang in Milli-Q-Wasser gequollen und anschließend die Masse des gequollenen Gels bestimmt (Mₛ).

Das Gel wurde im Ofen bei 37°C für 48h getrocknet und die Masse des trockenen Hydrogels bestimmt (M_{d}). Der Quellungsgrad (SR) wurde nach folgender Formel berechnet: $SR = \frac{{M}_{s} - {M}_{d}}{{M}_{d}}$

Die Experimente wurden dreimal durchgeführt. Mittelwerte und Standardabweichungen wurden mitgeteilt.

Der Quellungsgrad (SR) von 5% Thiol-MS-Gelen wurde in Wasser bei pH 7,0 gemessen. Es wurde eine Quellung von 33,6 mg Wasser/mg Polymer erhalten (siehe Tabelle 5). Thiol-VS-Gele zeigten ähnliche SR-Werte, während Thiol-Mal ca. 1,5-fach mehr anstieg. Diese Ergebnisse deuten auf einen ähnlichen Vernetzungsgrad von Thiol-MS- und Thiol-VS-Netzwerken und eine geringere Vernetzung von Thiol-Mal-Gelen hin.

Die hydrolytische Stabilität ist eine relevante Materialeigenschaft für Hydrogele, die in der 3D-Zellkultur verwendet werden.

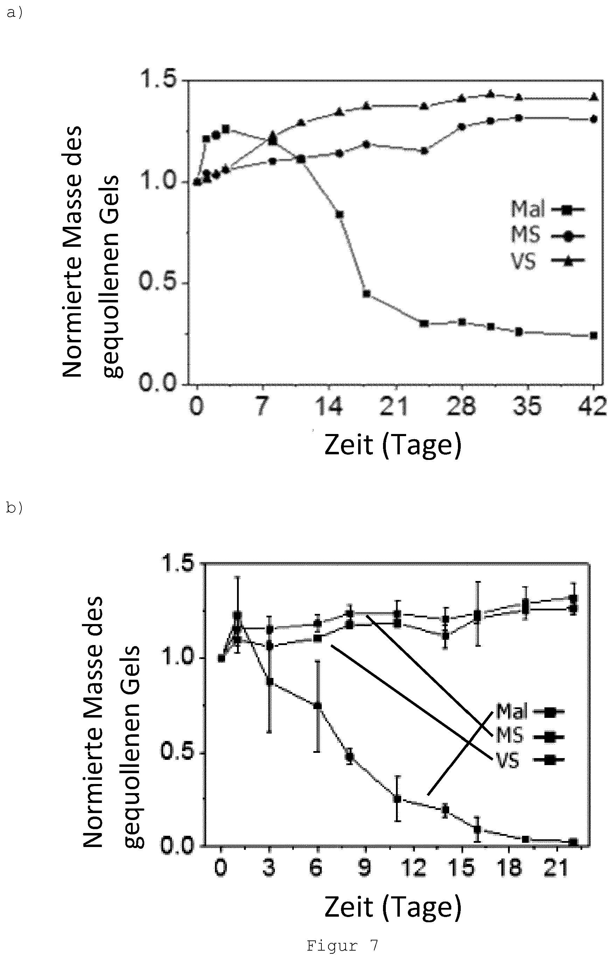

Daher wurde die hydrolytische Stabilität von 5 Gew.% Thiol-MS-Gelen durch gravimetrische Analyse des geschwollenen Gels nach der Inkubation im Zellkulturmedium bei 37°C für unterschiedliche Zeitpunkte über 4 Wochen bestimmt (Figur 7a). Die Masse der geschwollenen Thiol-MS-Gele erreichte in den ersten zwei Wochen das 1,2-fache der Ausgangsmasse, was auf geringe Gelerosion und hohe hydrolytische Stabilität der Thiol-MS-Gele hinweist. Es ist zu beachten, dass die Langzeitstabilität der Gele für die Langzeitzellkultur von Vorteil ist und eine Feinabstimmung der Abbaueigenschaften durch Copolymerisation mit spezifischen abbaubaren Sequenzen ermöglicht. [E. A. Phelps, N. O. Enemchukwu, V. F. Fiore, J. C. Sy, N. Murthy, T. A. Sulchek, T. H. Barker, A. J. Garcia, Advanced Materials 2012, 24, 64-70.] Die Stabilität des Thiol-MS-Systems war ähnlich wie bei Thiol-VS, das typischerweise für Langzeitkulturen verwendet wird, [M. P. Lutolf, G. P. Raeber, A. H. Zisch, N. Tirelli, J. A. Hubbell, Advanced Materials 2003, 15, 888-892.] und viel höher als bei Thiol-Mal-Gelen (1,2-fach in 2 Tagen und Hydrogelzerfall am Tag 18). [N. Boehnke, C. Cam, E. Bat, T. Segura, H. D. Maynard, Biomacromolecules 2015, 16,2101-2108.] Die Hydrolyse von Thiol-Mal-Gelen wird auf die geringe Stabilität der Thioether-Succinimid-Bindung zurückgeführt, die Retro-Michael- und Austauschreaktionen in Gegenwart anderer löslicher Thiole in Zellkulturmedien durchlaufen kann. Die Ergebnisse stimmen mit den Studien mit Modell-MS-Verbindungen überein, die eine überlegene Stabilität von thio-heteroaromatischen Konjugaten zeigen, die aus der Thiol-MS-Kopplung gegenüber Thiol-Mal-Verbindungen unter therapeutisch relevanten Bedingungen resultieren. [N. Toda, S. Asano, C. F. Barbas, Angew. Chem., Int. Ed. 2013, 52, 12592- 12596.] Schließlich zeigten Experimente, die mit Gelen mit 10 Gew.% durchgeführt wurden, dass Thiol-MS-Gele mehr als 6 Wochen hydrolytisch stabil blieben (siehe Figur 7b).

### Verwendung zur Zellverkapselung

### PEG Hydrogel-Präparation für 3D-Zellkultur

3D-PEG-Hydrogele wurden durch Anpassung des beschriebenen Protokolls hergestellt (Phelps et al Advanced Materials 2012, 24, 64-70; und Farrukh et al Adv. Funct. Mater. 2018). Die Vorläuferlösung von 20 kDa 4-Arm PEG Mal/VS/MS (100 mg mL⁻¹, 10% w/v) wurde durch Lösen im HEPES-Puffer (10 mM, pH 8,0) im sterilen Laminarstrom hergestellt. Lösungen von Cyclo(RGDfC) (3,45 mg mL⁻¹, 5 mM) und VPM-Peptid (GCRDVPMSMRGDRCG, 26,6 mg mL⁻¹, 15,68 mM) wurden ebenfalls im sterilen HEPES-Puffer (pH 8,0) zubereitet. Diese Konzentrationen wurden während aller Zellversuche konstant gehalten.

4-Arm-PEG Mal/MS/VS-Stammlösung (10% w/v) wurde im Volumenverhältnis 2:1 mit 5 mM cyclo(RGDfC) gemischt und für 30 min bei 37°C inkubiert. Die Zellsuspension (10x10⁻⁶ Zellen/mL⁻¹) im RPMI-Medium (2 µL) wurde der obigen Lösung zugegeben und 8 µL Tropfen der resultierenden Mischung wurden in je eine Ibidi 15-µ-Titerplatte Angiogenese-Dia gegeben. Sofort wurde die Lösung des VPM-Peptids (2 µL, 15,8 mM) in jede µ-Titerplatte gegeben, sorgfältig mit der Pipettenspitze vermischt und vernetzt. 15 Minuten lang wurde die Polymerisation der Mal- und MS-3D-Hydrogele durchgeführt, während VS-Hydrogele 45 Minuten lang bei 37 °C und 5% CO₂ polymerisiert wurden. Nach der Gelierung wurde das RPMI-Medium zugegeben und die Kultur für 1-3 Tage beibehalten. Alternativ wurde für die Sphäroidkultur das RPMI-Medium (2 µL) mit der cyclo(RGDfC)-modifizierten PEG-Vorläuferlösung (6 µL, wie oberhalb beschrieben) gemischt und jeweils (8 µL) in die µ-Titerplatte gegeben. Jeder Titerplatte wurde ein Fibringerinnsel hinzugefügt, gefolgt von der Zugabe von 15,8 mM VPM-Peptid (2 µL), das bei 37°C 15-45 min lang geliert werden konnte. Das Medium wurde jeder Titerplatte zugegeben und alle 24 Stunden während der Zellkultur durch frisches Medium ersetzt.

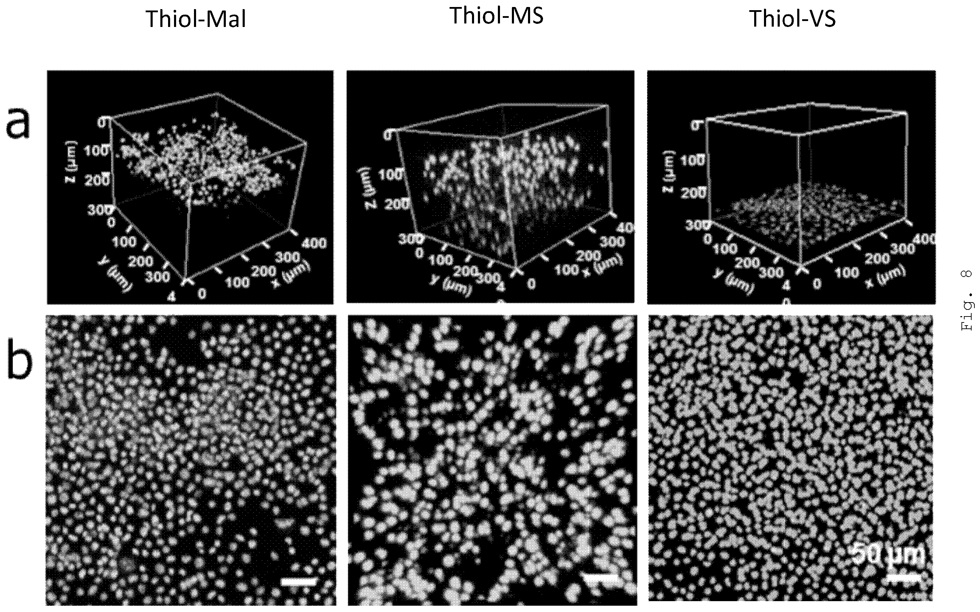

Bei diesem Verfahren wird die PEG-MS-Komponente zunächst mit dem cyclo(RGDfC)-Peptid funktionalisiert, dann mit L929-Fibroblasten vermischt und schließlich mit einem enzymatisch spaltbaren Dithiolpeptid (VPM) vernetzt. Eine Zusammensetzung von 4 Gew.% PEG-MS, 1 mM RGD-Peptid und 3,14 mM VPM wurden verwendet. [E. A. Phelps, N. O. Enemchukwu, V. F. Fiore, J. C. Sy, N. Murthy, T. A. Sulchek, T. H. Barker, A. J. Garcia, Advanced Materials 2012, 24, 64-70. A. Farrukh, J. I. Paez, A. del Campo, Advanced Functional Materials 2019, 29, 1807734.] Nach dem Mischen blieb die Lösung dünnflüssig, was eine Homogenisierung der Mischung durch Pipettieren bei geringen Scherkräften ermöglichte. Ein stabiles Gel bildete sich innerhalb von 15 min, sichtbar mit bloßem Auge. Die Verteilung der Zellen innerhalb des Hydrogels wurde mittels Z-Stapel-Imaging an einem Konfokalmikroskop analysiert. Eine gleichmäßige Verteilung der Zellen über die Dicke des Hydrogels wurde beobachtet (Figur 8a).

### Zellkulturbedingungen

Die Fibroblast L929 Zelllinie (ATCC) wurde bei 37 °C und 5% CO₂ im RPMI 1640 Medium (Gibco, 61870-010), ergänzt mit 10% FBS (Gibco, 10270) und 1% P/S (Invitrogen), kultiviert. Für suspendierte Zellkulturen wurden L929-Zellen (10×10⁶ Zellen mL⁻¹) direkt in der PEG-Vorläuferlösung während der Polymerisation suspendiert.

Für die Sphäroidenkultur wurde ein Fibringerinnsel der Zelllinie Fibroblast L929 mittels folgender Literaturberichte hergestellt. (J. L. West, Biomaterials 2008, 29, 2962-2968; C. A. DeForest, K. S. Anseth, Nature Chemistry 2011, 3, 925-931).

Zusammengefasst, wurde ein Pellet von 10×10⁶ Zellen mL⁻¹ in Fibrinogen dissoziiert (10 mg mL⁻¹ in PBS) und 2 µL Tropfen wurden auf einen hydrophoben, mit Sigmacote beschichteten Glasträger aufgebracht. 1 µL Thrombinlösung (5 UN mL⁻¹ in PBS) wurde zu jedem Tropfen Fibrinogen hinzugegeben und die Zellen wurden in einen Inkubator für 15 min gestellt, um ein Fibringerinnsel zu erhalten.

### Fixierung und Färbung

3D PEG-Hydrogel-Proben wurden mit 4%-iger PFA-Lösung für 2 h bei Raumtemperatur fixiert und mit PBS gewaschen. Die Proben wurden mit 1%-iger BSA-Lösung für 1 h blockiert, gefolgt von einer Permeabilisierung mit 0,5% Triton X-100 für 1 h. FITC-Phalloidin (1:200 in Wasser, Thermo Fisher Scientific) wurde zum Färben von Aktinfasern und DAPI (1:500 in Wasser, Life Technology) zum Färben von Kernen verwendet. Die Proben wurden für 5 h bei RT mit Antikörpern inkubiert und anschließend mit PBS gewaschen.

### Lebend-Tot-Assay

Das Zellkulturmedium wurde entfernt und die Proben wurden für 5 min mit Fluoreszeindiacetat (40 µg mL⁻¹) und Propidiumiodid (30 µg mL⁻¹) in PBS inkubiert. Die Proben wurden zweimal mit PBS gewaschen und mit dem Konfokalmikroskop Zeiss LSM 880 aufgenommen.

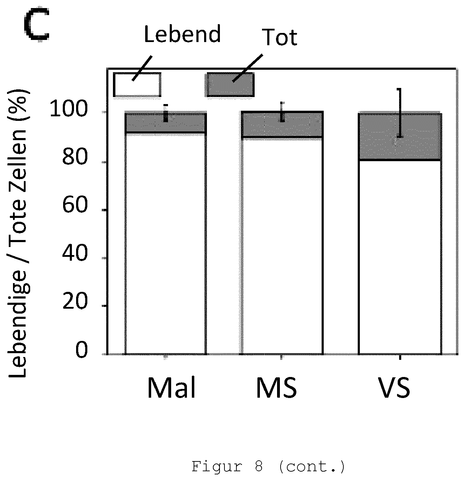

Lebend/Tot-Assays an Zellen, die für 1 Tag in Thiol-MS-Gelen verkapselt wurden, beweisen die Zytokompatibilität des erfindungsgemäßen Materials (>90% Lebensfähigkeit, Figur 8a, 8b, 8c). Diese Ergebnisse deuten darauf hin, dass die Vernetzungskinetik des Systems ideal ist, um homogene Konstrukte unter komfortablen und zytokompatiblen Versuchsbedingungen zu erhalten.

Umgekehrt führten Thiol-Mal-Hydrogele zu einer sofortigen Aushärtung beim Mischen von Vorläuferlösungen, was die korrekte Homogenisierung erschwerte und zu einer Zellagglomeration im oberen Teil des Gels führte. Andererseits ermöglichte das Thiol-VS-System eine gute Vermischung, aber die langsame Gelierungskinetik führte zu einer Zellsedimentation an der Unterseite des Gels. Diese Ergebnisse stehen im Einklang mit früheren Berichten von Peyton et al. über den Effekt der Vernetzungsrate bei der Verteilung von fluoreszierenden Perlen, die in Thiol-Mal-Hydrogelen verkapselt sind, [L. E. Jansen, L. J. Negrón-Piñeiro, S. Galarza, S. R. Peyton, Acta Biomaterialia 2018, 70, 120-128.] und von Shikanov et al., die auf die Notwendigkeit hinwiesen, Thiol-VS-Gele während der Aushärtung umzudrehen, um Zellablagerungen zu vermeiden. [J. Kim, Y. P. Kong, S. M. Niedzielski, R. K. Singh, A. J. Putnam, A. Shikanov, Soft Matter 2016, 12, 2076-2085.] In diesem Zusammenhang zeigen Thiol-MS-Hydrogele eine angemessenere Kinetik und überwinden diese Unannehmlichkeiten.

### Migrationsassay

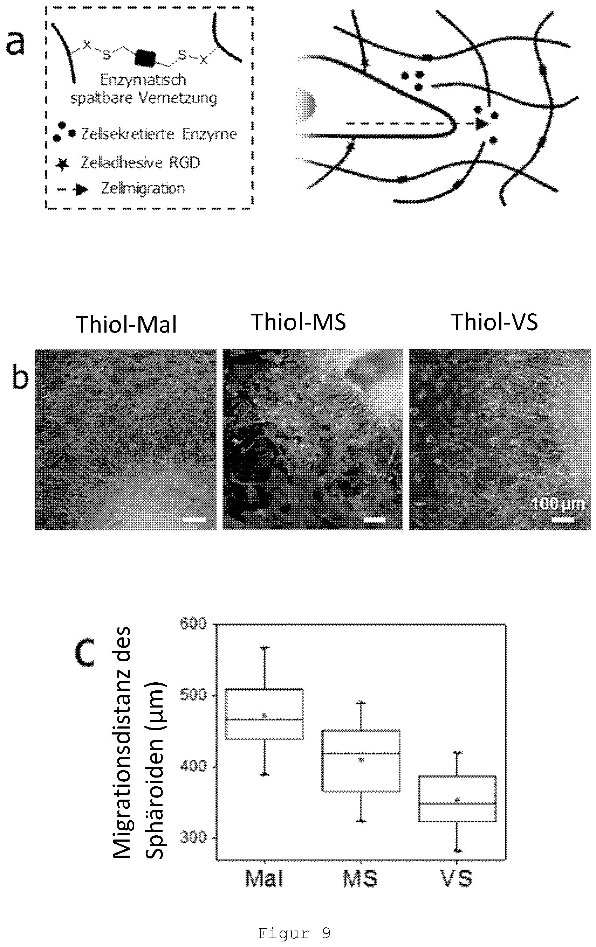

Um zu beweisen, dass Zellen, die in Thiol-MS-Hydrogelen gezüchtet wurden, funktionsfähig bleiben, wurde ein Migrationsassay durchgeführt. L929 Fibroblasten-Sphäroide wurden in den abbaubaren Thiol-MS-Hydrogelen verkapselt, [ A. Farrukh, J. I. Paez, A. del Campo, Advanced Functional Materials 2019, 29, 1807734.] 3 Tage lang kultiviert, fixiert und gefärbt. Der Zellmigrationsabstand vom Sphäroid wurde als Indikator für Degradation des Geles und Möglichkeit der Zelle zu Bewegung innerhalb des Geles quantifiziert (Figur 9a-c). Die Zellen legten eine Strecke von d ~425 µm zurück. Die Ergebnisse wurden mit den Ergebnissen verglichen, die für Thiol-Mal und Thiol-VS als Materialien für die 3D-Zellverkapselung erhalten wurden. Die Migrationsdistanz betrug ~470 µm für Mal und ~360 µm für VS-Systeme (Figur 9c). Dieses Ergebnis ist auf die Unterschiede im Vernetzungsgrad (G' _{37°C} = VS > MS > Mal, siehe Figur 3b)) und in der hydrolytischen Stabilität (MS = VS >> Mal, Figur 7) zurückzuführen. Eine geringere Vernetzung oder ein schnellerer Abbau schafft Freiraum für die Zellen, d.h. führt zu längeren Wanderwegen.

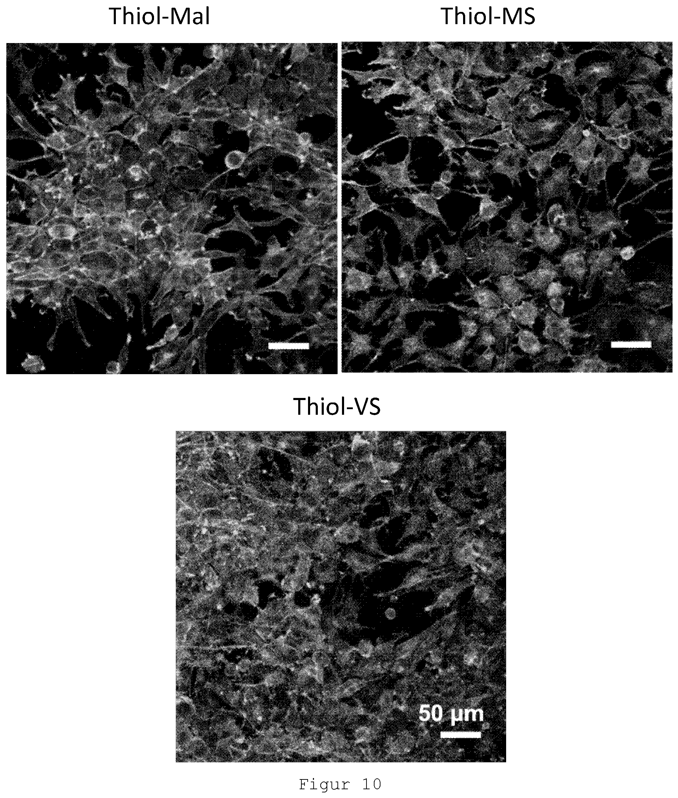

Weiterhin waren die in Thiol-MS-Hydrogelen kultivierten Zellen nach 3 Tagen Inkubation homogener im Gel verteilt und zeigten weniger Clusterbildungen als in den beiden anderen Systemen (siehe Figur 10).

Die Thiol-MS-Reaktion eignet sich zur Vernetzung von Hydrogelen bei der Zellverkapselung. Diese Reaktion erzielt Kinetiken zwischen Thiol-Mal- und Thiol-VS-Systemen und erreicht eine hohe Umwandlung. Die daraus resultierenden vernetzten Einheiten weisen eine gute hydrolytische Stabilität und Zytokompatibilität auf. Unter wässrigen milden Bedingungen ist die MS-Thiolreaktion orthogonal zu Alkoholen, Aminen, Carbonsäuren und Acrylat-Funktionsgruppen, [D. Zhang, N. O. Devarie-Baez, Q. Li, J. R. Lancaster, M. Xian, Organic Letters 2012, 14, 3396-3399. A. Farrukh, J. I. Paez, M. Salierno, A. del Campo, Angew. Chem. Int. Ed. 2016, 55, 2092- 2096. A. Farrukh, J. I. Paez, M. Salierno, W. Fan, B. Berninger, A. del Campo, Biomacromolecules 2017, 18, 906-913.] was die Anwendung dieses Vernetzungsmechanismus auf fast jedes natürliche polymere Grundgerüst von Interesse im biomedizinischen Bereich ermöglicht. Die Reaktivität des Thiol-MS-Paares kann durch den verwendeten pH und der Auswahl verschiedener MS-aromatischer Substrate reguliert werden. [N. Toda, S. Asano, C. F. Barbas, Angew. Chem., Int. Ed. 2013, 52, 12592- 12596.] Die Kombinationen all dieser Eigenschaften machen Thiol-MS zu einer überlegenen Alternative zu anderen reaktiven Chemikalien für die 3D-Zellverkapselung.

**Tabelle 1: Gelierungszeiten von verschiedenen Gelen gemessen in 10mM HEPES Puffer, T= 25°C, pH= 8-6,6**

| Gel | pH 8,0 | pH 7,5 | pH 7,0 | pH 6,6 |
|---|---|---|---|---|
| Thiol-Mal | < 1 s | 1-2 s | 2-3 s | 5-6 s |
| Thiol-MS | 3 s | 6 s | 12 s | 3,5 Min |
| Thiol-VS | 8 Min | 22 Min | 88 Min | 190 Min |

**Tabelle 2: Berichtete Reaktionsraten zweiter Ordnung für ausgewählte nukleophile Thiol-X-Kopplungen unter milden wässrigen Bedingungen.**

| X | Reaktionsrate k₂ (M⁻¹S⁻¹) | Quelle |
|---|---|---|
| Mal | 734, 0 | F. Saito, H. Noda, J. W. Bode, ACS Chemical Biology 2015, 10, 1026-1033. |
| MS | 0,4-16,0 | X. Chen, H. Wu, C.-M. Park, T. H. Poole, G. Keceli, N. O. Devarie-Baez, A. W. Tsang, W. T. Lowther, L. B. Poole, S. B. King, M. Xian, C. M. Furdui, ACS Chemical Biology 2017, 12, 2201-2208. |
| VS | 0,08-1,0 | H. Wang, F. Cheng, M. Li, W. Peng, J. Qu, Langmuir 2015, 31, 3413-3421. |

**Tabelle 3: Ermittelte Gelierungszeit im Bulk für Thiol-MS Hydrogele bei unterschiedlichen Temperaturen (5 Gew.% in 10 mM HEPES-Puffer).**

| Gel | T: 45 °C | 25 °C | 15 °C | 5 °C |
|---|---|---|---|---|
| Thiol-MS (pH 7, 0) | 7 s | 12 s | 22 s | 30 s |

**Tabelle 4: Ermittelte Gelierungszeit im Bulk für Thiol-MS Hydrogele bei unterschiedlichem Polymergehalt (10 mM HEPES-Puffer, pH 7, 5, T = 25 °C)**

| Gel | 10, 0 Gew.% | 7,5 Gew.% | 5,0 Gew.% | 2,5 Gew.% | 1, 3 Gew.% |
|---|---|---|---|---|---|
| Thiol-MS | 2-3 s | 4 s | 6 s | 10 s | 18 s |

**Tabelle 5: Quellverhältnis in Wasser von Thiol-X-Hydrogelen (5 Gew.% Polymeranteil; n= 3).**

| | Quellverhältnis | |
|---|---|---|
| Gel | [mg Wasser / mg Gel] | Relativer Wert |
| Thiol-Mal | 48,8 ± 4,3 | 1,45 |
| Thiol-MS | 33,6 ± 1,0 | 1,00 |
| Thiol-VS | 35,3 ± 2,8 | 1,05 |

## Patentansprüche

1. Verfahren zur Herstellung eines Hydrogels umfassend folgende Schritte:
a) Herstellen einer Zusammensetzung umfassend
a1) mindestens ein Makromer umfassend als funktionelle Gruppen mindestens zwei Thiolgruppen,
a2) mindestens ein Makromer umfassend als funktionelle Gruppen mindestens zwei aromatische oder heteroaromatische Gruppen, die jeweils mit mindestens einer Sulfonylgruppe substituiert sind, wobei mindestens eine Komponente a1) oder a2) mindestens drei der genannten funktionellen Gruppen aufweist;
b) Reaktion der beiden Makromere über die funktionellen Gruppen unter Bildung eines Hydrogels, wobei Reaktionsbedingungen gewählt werden, bei welchen die Thiolgruppen des ersten Makromers a1) eine nukleophile aromatische Substitution an der Gruppe Ar durchführen können, wobei die Gruppe SO₂-R¹ als Abgangsgruppe dient;
wobei die funktionellen Gruppen des Makromers a2) funktionelle Gruppen der Formel (1) sind:
M-Ar-SO₂-R¹ (1)
wobei folgendes gilt:
Ar steht für eine elektronenarme Arylgruppe mit 6 bis 40 C-atomen oder elektronenarme Heteroarylgruppe mit 1 bis 40 C-Atomen und mindestens ein Heteroatom mit der Maßgabe, dass die Summe aus C-Atomen und Heteroatomen mindestens 5 ergibt, wobei die elektronenziehenden Gruppen und/oder Heteroatome in Konjugation zur Abgangsgrupppe -SO₂-R¹ stehen, und wobei Ar ausgewählt ist aus der Gruppe umfassend Nitrobenzole, Benzaldehyde, Benzonitrile, Benzoesäureester, Pyridine, Pyrimidine, Pyrazine, Pyridazine, Triazine, Tetrazine, Oxazole, Isooxazol, Thiazole, Isothiazol, Oxadiazole, Thiadiazole, wie 1,2,3-Thiadiazol, 1,2,4-Thiadiazol, 1,2,5-Thiadiazol oder 1,3,4-Thiadiazol, Imidazol, Pyrazol, Triazole, Tetrazol, Chinoline, Isochinoline, Benzimidazol, Benzoxazol, Benzothiazol, Benzopyridazin, Benzpyrimidin, Chinoxalin, Benzotriazol, Naphthalimid, Purin, Pteridin, Indolizin und Benzothiadiazol, wobei Ar jeweils noch mit einer oder mehrerer Gruppen R² substituiert sein kann;
M steht für eine kovalente Verbindung zum Makromer;
R¹ steht für N(R²)₂, eine geradkettige Alkylgruppe mit 1 bis 20 C-Atomen oder eine verzweigte oder cyclische Alkylgruppe mit 3 bis 20 C-Atomen, oder eine Alkenyl- oder Alkinylgruppe mit 2 bis 20 C-Atomen, wobei die Alkyl-, Alkenyl- oder Alkinylgruppe jeweils mit einem oder mehreren Resten R² substituiert sein kann und wobei eine oder mehrere nicht benachbarte CH₂-Gruppen durch O, NR², S, R²C=CR², C=C, C=O, C(=O)O oder C(=O)NR² ersetzt sein können, oder eine Arylgruppe oder Heteroarylgruppe, die jeweils mit einem oder mehreren Resten R² substituiert sein kann.
R² ist bei jedem Auftreten gleich oder verschieden H, D, F, Cl, Br, I, N(R³)₂, CN, NO₂, OR³, SR³, C(=O)OR³, C(=O)N(R³)₂, C(=O)R³, eine geradkettige Alkylgruppe mit 1 bis 20 C Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R³ substituiert sein kann, wobei eine oder mehrere nicht benachbarte CH₂-Gruppen durch R³C=CR³, C=C, C=O, NR³, O, S, C(=O)O oder C(=O)NR³ ersetzt sein können, oder eine Arylgruppe oder Heteroarylgruppe, die jeweils mit einem oder mehreren Resten R³ substituiert sein kann.
R³ ist bei jedem Auftreten gleich oder verschieden H, D, F, OH, oder ein aliphatischer, aromatischer und/oder heteroaromatischer organischer Rest, insbesondere eine geradkettige Alkylgruppe mit 1 bis 20 C-Atomen, in dem auch ein oder mehrere H Atome durch F ersetzt sein können.

2. Verfahren nach Anspruch 1, **dadurch gekennzeichnet, dass** das Makromer eine mittlere molare Masse von unter 500 kDa aufweist, wobei die mittlere molare Masse als gewichtsmittleres Molekulargewicht mit Gel-Permeations-Chromatographie (GPC) bestimmt wird.

3. Verfahren nach einem der Ansprüche 1 oder 2, **dadurch gekennzeichnet, dass** die Makromere 2, 3, 4, 5, 6, 7, 8, 9 oder 10 funktionelle Gruppen aufweisen.

4. Verfahren nach einem der Ansprüche 1 bis 3, **dadurch gekennzeichnet, dass** die Makromere auf Oligomeren oder Polymeren, wie Poly(meth)acrylate wie Poly(meth)acrylamide, Po-ly(meth)acrylsäure, PolyHPMA oder PolyHEMA, Polyethylenglykol (PEG), Polyvinylalkohol (PVA), Polyurethan (PU), Polyvinylpyrrolidon (PVP), Polyamide, Poly(amidoamine) (PAMAM), Polyester, Polylactide, Polyglycolsäure (PGA) oder Poly(lactid-co-glycolid) (PLGA), Polyanhydride, Poly(ortho)ester, Polyacetale, Poloxamere (Blockcopolymere aus Ethylenoxid (PEG) und Propylenoxid (PPG)) wie PEG-Co-PPG-Co-PEG), Poly-2-oxazoline, Polyphosphazene, Polyglycerin, Polyamine wie Polylysin oder Polyethylenimin (PEI), Polycarbonate, Polyglutaminsäure, insbesondere Poly-Gamma-Glutaminsäure, Polyasparaginsäure (PASA), Polyphosphonate, DNA, RNA, Gelatine, Polyhydroxyalkanoate (PHA), Proteine oder Peptide wie Kollagene, VPM, Albumin oder Fibrin, Polysacccharide wie Agarose, Chitin, Chitosan, Chondroitin, Mannan, Inulin, Dextran, Cellulose, Alginate oder Hyaluronsäure, basieren.

5. Verfahren nach einem der Ansprüche 1 bis 4, **dadurch gekennzeichnet, dass** Ar für eine 1,3,4-Oxadiazolgruppe steht, welche mit einer Phenylgruppe substituiert ist.

6. Verfahren nach einem der Ansprüche 1 bis 5, **dadurch gekennzeichnet, dass** der Gehalt an Makromer in der Zusammensetzung bei 1 bis 30 Gew.% liegt.

7. Verfahren nach einem der Ansprüche 1 bis 6, **dadurch gekennzeichnet, dass** die Gelierung bei physiologischen Bedingungen stattfindet.

8. Hydrogel erhalten nach einem der Ansprüche 1 bis 7.

9. Zusammensetzung zur Herstellung eines Hydrogels umfassend die Komponenten a1) und a2) nach einem der Ansprüche 1 bis 7.

10. Kit zur Herstellung eines Hydrogels umfassend die Komponenten a1) und a2) nach einem der Ansprüche 1 bis 6.

11. Verwendung eines Hydrogels nach Anspruch 8 zur Verkapselung von Zellen, für dreidimensionale Zellkulturen, Organoide, Biomaterialien, injizierbare Biomaterialien, Zelltherapien, Gewebemodifizierung, Geweberegeneration, Gewebetransplantation, regenerative Medizin, 3D Druck, 3D Bioprinting, Wundverbände oder Wundbehandlung, Transportmittel für Wirkstoffe, In-vitro-Modelle zum Untersuchen oder Testen von Diagnostika oder Therapeutika oder Zelltransplantationen.

12. Verfahren zur Modifikation von Gelen, umfassend die Schritte:
a) Bereitstellen eines Gels, umfassend mindestens zwei funktionelle Gruppen gemäß Komponente a1) oder mindestens zwei funktionelle Gruppen gemäß Komponente a2);
b) Zugabe einer Zusammensetzung umfassend mindestens ein Makromer nach einem der Ansprüche 1 bis 6 gemäß der jeweils anderen Komponente, wobei das Makromer mindestens zwei funktionelle Gruppen aufweist;
c) Modifikation des Gels durch Reaktion der funktionellen Gruppen des Makromers mit dem Gel, wobei die Reaktionsbedingungen nach Anspruch 1 gewählt werden.

## Claims

1. A process for preparing a hydrogel, comprising the following steps:
a) preparing a composition comprising
a1) at least one macromer comprising as functional groups at least two thiol groups,
a2) at least one macromer comprising as functional groups at least two aromatic or heteroaromatic groups each substituted by at least one sulfonyl group, where at least one component a1) or a2) contains at least three of the stated functional groups;
b) reacting the two macromers via the functional groups to form a hydrogel, wherein reaction conditions are se- lected, under which the thiol groups of the first macromer are able to perform a nucleophilic aromatic substitution on the group Ar, with the group SO₂-R¹ serving as leaving group, wherein the functional groups of the macromer a2) are func-
tional groups of the formula (1):
M-Ar-SO₂-R¹ (1)
where:
Ar is an electron-deficient aryl group with 6 to 40 C atoms or electron-deficient heteroaryl group with 1 to 40 C atoms and at least one heteroatom, with the proviso that the sum total of C atoms and heteroatoms makes at least 5, wherein the electron-withdrawing groups and/or hetero atoms are in conjugation with the leaving group -SO₂-R¹, and wherein Ar is selected from the group encompassing nitrobenzenes, benzaldehydes, benzonitriles, benzoic esters, pyridines, pyrimidines, pyrazines, pyridazines, triazines, tetrazines, oxazoles, isoxazole, thiazoles, isothiazole, oxadiazoles, thiadiazoles, such as 1,2,3-thiadiazole, 1,2,4-thiadiazole, 1,2,5-thiadiazole or 1,3,4-thiadiazole, imidazole, pyrazole, triazoles, tetrazole, quinolines, isoquinolines, benzimidazole, benzoxazole, benzothiazole, benzopyridazine, benzopyrimidine, quinoxaline, benzotriazole, naphthalimide, purine, pteridine, indolizine and benzothiadiazole, where Ar may be substituted in each case additionally by one or more groups R²,
M is a covalent connection to the macromer;
R¹ is N(R²)₂, a straight-chain alkyl group having 1 to 20 C atoms or a branched or cyclic alkyl group having 3 to 20 C atoms, or an alkenyl or alkynyl group having 2 to 20 C atoms, where the alkyl, alkenyl or alkynyl group may be substituted in each case by one or more radicals R² and where one or more nonadjacent CH₂ groups may be replaced by O, NR², S, R²C=CR², C≡C, C=O, C(=O)O or C(=O)NR², or is an aryl group or heteroaryl group which may be substituted in each case by one or more radicals R².
R², identical or different at each occurrence, is H, D, F, Cl, Br, I, N(R³)₂, CN, NO₂, OR³, SR³, C(=O)OR³ C(=O)N(R³)₂, C(=O)R³, a straight-chain alkyl group having 1 to 20 C atoms or an alkenyl or alkynyl group having 2 to 20 C atoms or a branched or cyclic alkyl group having 3 to 20 C atoms, where the alkyl, alkenyl or alkynyl group may be substituted in each case by one or more radicals R³, where one or more nonadjacent CH₂ groups may be replaced by R³C=CR³, C≡C, C=O, NR³, O, S, C(=O)O or C(=O)NR³, or is an aryl group or heteroaryl group which may be substituted in each case by one or more radicals R³.
R³, identical or different at each occurrence, is H, D, F, OH, or an aliphatic, aromatic and/or heteroaromatic organic radical, more particularly a straight-chain alkyl group having 1 to 20 C atoms, in which one or more H atoms may also be replaced by F.

2. The process as claimed in claim 1, **characterized in that** the macromer has an average molar mass of less than 500 kDa, wherein the average molar mass is determined as weight-average molecular weight by gel permeation chromatography (GPC).

3. The process as claimed in either of claims 1 and 2, **characterized in that** the macromers contain 2, 3, 4, 5, 6, 7, 8, 9 or 10 functional groups.

4. The process as claimed in any of claims 1 to 3, **characterized in that** the macromers are based on oligomers or polymers, such as poly(meth)acrylates such as poly(meth)acrylamides, poly(meth)acrylic acid, polyHPMA or polyHEMA, polyethylene glycol (PEG), polyvinyl alcohol (PVA), polyurethane (PU), poly¬vinylpyrrolidone (PVP), polyamides, poly(amido¬amines) (PAMAM), polyesters, polylactides, poly¬glycolic acid (PGA) or poly(lactide-co-glycolide) (PLGA), polyanhydrides, poly(ortho)esters, poly¬acetals, poloxamers (block copolymers of ethylene oxide (PEG) and propylene oxide (PPG)) such as PEG-Co-PPG-Co-PEG), poly-2 oxazolines, polyphospha-zenes, polyglycerol, polyamines such as polylysine or polyethylenimine (PEI), polycarbonates, poly¬glutamic acid, more particularly poly-gamma-glutamic acid, polyaspartic acid (PASA), polyphos¬phonates, DNA, RNA, gelatin, polyhydroxyalkanoates (PHA), proteins or pep¬tides such as collagens, VPM, albumin or fibrin, polysaccharides such as agarose, chitin, chitosan, chondroitin, mannan, inulin, dextran, cellulose, alginates or hyaluronic acid.

5. The process as claimes in any of the claims 1 to 4, **characterized in that** Ar is a 1,3,4-oxadiazole group, which is substituted by a phenyl group.

6. The process as claimed in any of claims 1 to 6, **characterized in that** the macromer content of the composition is 1 to 30 wt%.

7. The process as claimed in any of claims 1 to 7, **characterized in that** the gelling takes place under physiological conditions.

8. A hydrogel obtained as claimed in any of claims 1 to 7.

9. A composition for preparing a hydrogel, comprising components a1) and a2) as claimed in any of claims 1 to 7.

10. A kit for preparing a hydrogel, comprising compo¬nents a1) and a2) as claimed in any of claims 1 to 6.

11. The use of a hydrogel as claimed in claim 8 for encapsu¬lating cells, for three-dimensional cell cultures, organoids, biomaterials, injectable biomaterials, cell therapies, tissue modification, tissue regeneration, tissue transplantation, regenerative medicine, 3D printing, 3D bioprinting, wound dressings or wound treatment, transport agents for active ingredients, invitro models for studying or testing diagnostic or therapeutic agents, or cell trans¬plantations.

12. A process for modifying gels, comprising the steps of:
a) providing a gel thereof, comprising at least two functional groups as per component a1) or at least two functional groups as per component a2);
b) adding a composition comprising at least one macromer as claimed in any of claims 1 to 6 as per the respectively other component, where the macromer contains at least two functional groups;
c) modifying the gel or the precursor thereof by reacting the functional groups of the macromer with the gel, wherein the reaction conditions are selected according to claim 1.

## Revendications

1. Procédé pour la préparation d'un hydrogel, comprenant les étapes suivantes :
a) préparation d'une composition comprenant
a1) au moins un macromère comprenant, en tant que groupes fonctionnels, au moins deux groupes thiol,
a2) au moins un macromère comprenant, en tant que groupes fonctionnels, au moins deux groupes aromatiques ou hétéroaromatiques qui sont à chaque fois substitués par au moins un groupe sulfonyle, au moins un composant a1) ou a2) présentant au moins trois des groupes fonctionnels mentionnés ;
b) réaction des deux macromères par l'intermédiaire des groupes fonctionnels avec formation d'un hydrogel, des conditions de réaction dans lesquelles les groupes thiol du premier macromère a1) peuvent effectuer une substitution aromatique nucléophile sur le groupe Ar étant choisies, le groupe SO₂-R¹ servant de groupe partant ;
les groupes fonctionnels du macromère a2) étant des groupes fonctionnels de formule (1) :
M-Ar-SO₂-R¹ (1)
dans laquelle :
Ar représente un groupe aryle pauvre en électrons comprenant 6 à 40 atomes de carbone ou un groupe hétéroaryle pauvre en électrons comprenant 1 à 40 atomes de carbone et au moins un hétéroatome, sous réserve que la somme des atomes de carbone et des hétéroatomes vaille au moins 5, les groupes et/ou hétéroatomes attracteurs d'électrons étant en conjugaison avec le groupe partant -SO₂-R¹ et Ar étant choisi dans le groupe comprenant les nitrobenzènes, les benzaldéhydes, les benzonitriles, les esters de l'acide benzoïque, les pyridines, les pyrimidines, les pyrazines, les pyridazines, les triazines, les tétrazines, les oxazoles, l'isooxazole, les thiazoles, l'isothiazole, les oxadiazoles, les thiadiazoles, tels que le 1,2,3-thiadiazole, le 1,2,4-thiadiazole, le 1,2,5-thiadiazole ou le 1,3,4-thiadiazole, l'imidazole, le pyrazole, les triazoles, le tétrazole, la quinoléine, l'isoquinoléine, le benzimidazole, le benzoxazole, le benzothiazole, la benzopyridazine, la benzopyrimidine, la quinoxaline, le benzotriazole, le naphtalimide, la purine, la ptéridine, l'indolizine et le benzothiadiazole, Ar pouvant à chaque fois encore être substitué par un ou plusieurs groupes R² ;
M représente une liaison covalente avec le macromère ;
R¹ représente N(R²)₂, un groupe alkyle linéaire comprenant 1 à 20 atomes de carbone ou un groupe alkyle ramifié ou cyclique comprenant 3 à 20 atomes de carbone ou un groupe alcényle ou alcynyle comprenant 2 à 20 atomes de carbone, le groupe alkyle, alcényle ou alcynyle pouvant à chaque fois être substitué par un ou plusieurs radicaux R² et un ou plusieurs groupes CH₂ non adjacents pouvant être replacés par O, NR², S, R²C=CR², C=C, C=O, C(=O)O ou C(=O)NR², ou un groupe aryle ou hétéroaryle, qui peut à chaque fois être substitué par un ou plusieurs radicaux R²,
R² représente, en chaque occurrence, de manière identique ou différente, H, D, F, Cl, Br, I, N(R³)₂, CN, NO₂, OR³, SR³, C(=O)OR³, C(=O)N(R³)₂, C(=O)R³, un groupe alkyle linéaire comprenant 1 à 20 atomes de carbone ou un groupe alcényle ou alcynyle comprenant 2 à 20 atomes de carbone ou un groupe alkyle ramifié ou cyclique comprenant 3 à 20 atomes de carbone, le groupe alkyle, alcényle ou alcynyle pouvant à chaque fois être substitué par un ou plusieurs radicaux R³, un ou plusieurs groupes CH₂ non adjacents pouvant être remplacés par R³C=CR³, C=C, C=O, NR³, O, S, C(=O)O ou C(=O)NR³, ou un groupe aryle ou hétéroaryle, qui peut à chaque fois être substitué par un ou plusieurs radicaux R³,
R³ représente, en chaque occurrence, de manière identique ou différente, H, D, F, OH ou un radical organique aliphatique, aromatique et/ou hétéroaromatique, en particulier un groupe alkyle linéaire comprenant 1 à 20 atomes de carbone, dans lequel un ou plusieurs atomes H peuvent également être remplacés par F.

2. Procédé selon la revendication 1, **caractérisé en ce que** le macromère présente une masse moléculaire moyenne inférieure à 500 kDa, la masse moléculaire moyenne étant déterminée en tant que poids moléculaire moyen en poids à l'aide de la chromatographie par perméation de gel (CPG).

3. Procédé selon l'une des revendications 1 ou 2, **caractérisé en ce que** les macromères présentent 2, 3, 4, 5, 6, 7, 8, 9 ou 10 groupes fonctionnels.

4. Procédé selon l'une des revendications 1 à 3, **caractérisé en ce que** les macromères sont à base d'oligomères ou de polymères, tels que les poly(méth)acrylates, tels que les poly(méth)acrylamides, le poly(acide (méth)acrylique), le polyHPMA ou le polyHEMA, le polyéthylèneglycol (PEG), le poly(alcool vinylique) (PVA), le polyuréthane (PU), la polyvinylpyrrolidone (PVP), les polyamides, les poly(amidoamines) (PAMAM), les polyesters, les polylactides, le poly(acide glycolique) (PGA) ou le poly(lactide-co-glycolide) (PLGA), les polyanhydrides, les poly(ortho)esters, les polyacétals, les poloxamères (copolymères à blocs d'oxyde d'éthylène (PEG) et d'oxyde de propylène (PPG) tels que le PEG-co-PPG-co-PEG), les poly-2-oxazolines, les polyphosphazènes, le polyglycérol, les polyamines telles que la polylysine ou la polyéthylène-imine (PEI), les polycarbonates, le poly(acide glutamique), en particulier le poly(acide gamma-glutamique), le poly(acide aspartique) (PASA), les polyphosphonates, l'ADN, l'ARN, la gélatine, les polyhydroxyalcanoates (PHA), les protéines ou les peptides tels que le collagène, le VPM, l'albumine ou la fibrine, les polysaccharides tels que l'agarose, la chitine, le chitosane, la chondroïtine, le mannane, l'inuline, le dextrane, la cellulose, les alginates ou l'acide hyaluronique.

5. Procédé selon l'une des revendications 1 à 4, **caractérisé en ce qu'**Ar représente un groupe 1,3,4-oxadiazole qui est substitué par un groupe phényle.

6. Procédé selon l'une des revendications 1 à 5, **caractérisé en ce que** la teneur en macromère dans la composition est de 1 à 30% en poids.

7. Procédé selon l'une des revendications 1 à 6, **caractérisé en ce que** la gélification a lieu dans des conditions physiologiques.

8. Hydrogel obtenu selon l'une des revendications 1 à 7.

9. Composition pour la préparation d'un hydrogel comprenant les composants a1) et a2) selon l'une des revendications 1 à 7.

10. Kit pour la préparation d'un hydrogel comprenant les composants a1) et a2) selon l'une des revendications 1 à 6.

11. Utilisation d'un hydrogel selon la revendication 8 pour l'encapsulation de cellules, pour les cultures cellulaires tridimensionnelles, les organoïdes, les biomatériaux, les biomatériaux injectables, les thérapies cellulaires, la modification tissulaire, la régénération tissulaire, la transplantation tissulaire, la médecine régénérative, l'impression 3D, la bio-impression 3D, les pansements ou le traitement de plaies, les moyens de transport pour des principes actifs, les modèles in vitro pour l'étude ou le test d'agents diagnostiques ou les produits thérapeutiques ou les transplantations cellulaires.

12. Procédé de modification de gels, comprenant les étapes :
a) mise à disposition d'un gel comprenant au moins deux groupes fonctionnels selon le composant a1) ou au moins deux groupes fonctionnels selon le composant a2) ;
b) ajout d'une composition comprenant au moins un macromère selon l'une des revendications 1 à 6 conformément à l'autre composant respectif, le macromère présentant au moins deux groupes fonctionnels ;
c) modification du gel par réaction des groupes fonctionnels du macromère avec le gel, les conditions de réaction selon la revendication 1 étant choisies.
